# EUROPEAN PATENT SPECIFICATION

(11) **EP 3 307 906 B1**
(45) Date of publication and mention of the grant of the patent: **14.10.2020**
(21) Application number: 16732413.6
(22) Date of filing: 09.06.2016
(51) Int. Cl.: C12Q 1/6809

(54) **METHODS FOR DETECTING RISK OF HAVING A BLOODSTREAM INFECTION**
VERFAHREN ZUR ERKENNUNG DER GEFAHR EINER BLUTSTROMINFEKTION
MÉTHODES POUR DÉTECTER LE RISQUE DE PRÉSENTER UNE BACTÉRIÉMIE

(30) Priority: 09.06.2015 US 201562172986 P
(43) Date of publication of application: 18.04.2018
(73) Proprietor: Regents of the University of Minnesota, Minneapolis, MN 55455-2020 (US); Centre Hospitalier Universitaire De Nantes, 44093 Nantes Cedex 1 (FR)
(72) Inventor: KNIGHTS, Dan, Saint Paul, MN 55108 (US); MONTASSIER, Emmanuel, 44000 Nantes (FR)
(74) Representative: Potter Clarkson
(86) International application number: PCT/US2016/036612
(87) International publication number: WO 2016/201053

(56) References cited:
- WO-A1-2014/070014
- WO-A2-2004/017925
- Y. TAUR ET AL: "Intestinal Domination and the Risk of Bacteremia in Patients Undergoing Allogeneic Hematopoietic Stem Cell Transplantation", CLINICAL INFECTIOUS DISEASES, vol. 55, no. 7, 1 October 2012 (2012-10-01), pages 905-914, XP055297806, US ISSN: 1058-4838, DOI: 10.1093/cid/cis580
- ANYA LEVINSON ET AL: "Acute Gastrointestinal Graft-vs-Host Disease Is Associated With Increased Enteric Bacterial Bloodstream Infection Density in Pediatric Allogeneic Hematopoietic Cell Transplant Recipients", CLINICAL INFECTIOUS DISEASES, vol. 61, no. 3, 5 May 2015 (2015-05-05), pages 350-357, XP055298288, US ISSN: 1058-4838, DOI: 10.1093/cid/civ285
- Y. TOUCHEFEU ET AL: "Systematic review: the role of the gut microbiota in chemotherapy- or radiation-induced gastrointestinal mucositis - current evidence and potential clinical applications", ALIMENTARY PHARMACOLOGY & THERAPEUTICS., 11 July 2014 (2014-07-11), pages n/a-n/a, XP055297816, GB ISSN: 0269-2813, DOI: 10.1111/apt.12878
- EMMANUEL MONTASSIER ET AL: "Pretreatment gut microbiome predicts chemotherapy-related bloodstream infection", GENOME MED, vol. 11, no. 1, 28 April 2016 (2016-04-28) , page 210, XP055298268, London, UK ISSN: 1756-994X, DOI: 10.1186/s13073-016-0301-4
- Y. TAUR ET AL: "The effects of intestinal tract bacterial diversity on mortality following allogeneic hematopoietic stem cell transplantation", BLOOD, vol. 124, no. 7, 14 August 2014 (2014-08-14), pages 1174-1182, XP055252479, US ISSN: 0006-4971, DOI: 10.1182/blood-2014-02-554725
- MICHEL J. VAN VLIET ET AL: "The Role of Intestinal Microbiota in the Development and Severity of Chemotherapy-Induced Mucositis", PLOS PATHOGENS, vol. 6, no. 5, 27 May 2010 (2010-05-27), page e1000879, XP055297804, DOI: 10.1371/journal.ppat.1000879
- LUDMILA KHAILOVA ET AL: "Probiotic Administration Reduces Mortality and Improves Intestinal Epithelial Homeostasis in Experimental Sepsis", ANESTHESIOLOGY., vol. 119, no. 1, 1 July 2013 (2013-07-01), pages 166-177, XP055298331, PHILADELPHIA, PA, US ISSN: 0003-3022, DOI: 10.1097/ALN.0b013e318291c2fc
- C. UBEDA ET AL: "Intestinal Microbiota Containing Barnesiella Species Cures Vancomycin-Resistant Enterococcus faecium Colonization", INFECTION AND IMMUNITY, vol. 81, no. 3, 1 March 2013 (2013-03-01), pages 965-973, XP055386852, US ISSN: 0019-9567, DOI: 10.1128/IAI.01197-12

## Description

### BACKGROUND

Hematopoietic stem cell transplantation (HSCT) is commonly applied as a curative treatment in patients with hematological malignancy. The therapy includes the administration of high-dose chemotherapy for transplantation conditioning, followed by the intravenous transfusion of marrow (Tuncer et al., World J. Gastroenterol. WJG 2012;18(16):1851-1860). A frequent toxicity of myeloablative doses of chemotherapy used during the HSCT procedure is gastro-intestinal (GI) mucositis, defined as inflammatory and ulcerative lesions of the GI tract responsible for mucosal injury (Peterson et al., Ann. Oncol. 2011;22 Suppl 6:vi78-84). Globally, GI mucositis has been reported to affect up to 80% of all cancer patients, but the incidence is highly dependent on the chemotherapy regimen (Keefe et al. Cancer 2007;109(5):820-831).

A current model, introduced by Sonis, described the GI mucositis pathogenesis (Sonis, Nat. Rev. Cancer 2004;4(4):277-284). It includes an ulcerative phase with increased permeability and damage to the intestinal mucosal barrier. This promotes bacterial translocation, defined as the passage of bacteria from the GI tract to extra-intestinal sites, such as the bloodstream (Berg, Adv. Exp. Med. Biol. 1999;473:11-30). Thus, bacteremia, or bloodstream infection (BSI), remains a common life-threatening complication with well-documented morbidity and mortality in patients with cancer (Almyroudis et al. Transpl. Infect. Dis. 2005;7(1):11-17; Blennow et al., Transpl. Infect. Dis. 2014;16(1):106-114; Mikulska et al. Clin. Infect. Dis. 2012;55(12):1744). BSI is particularly frequent during the early transplant period due to the intensive chemotherapy regimen administered prior to HSCT (Elting et al., Clin. Infect. Dis.. 1997;25(2):247-259; Freifeld et al. Clin. Infect. Dis. 2011;52(4):e56-93; Klastersky, Clin. Infect. Dis. 2004;39 Suppl 1:S32-37).

Whereas the model of pathobiology of mucositis reported above is silent on the role of the intestinal microbiome, Van Vliet et al. proposed that the five pathways described by Sonis might potentially be influenced by the intestinal microbiome (Van Vliet et al., PLoS Pathog. 2010;6(5):e1000879). Thus, alterations of the intestinal microbiome might be involved in the development of subsequent BSI. A previous study reported that intestinal domination, defined as occupation of at least 30% of the microbiota by a single bacterial taxon, is associated with BSI in patients undergoing allo-HSCT. Indeed, patients with enterococcal domination had a 9-fold (95% CI: 2-45) increased risk of Vancomycin-resistant *Enterococcus* (VRE) BSI relative to patients without enterococcal domination, and patients with domination by Proteobacteria had a 5-fold (95% CI: 1-20) increased risk of BSI with gram-negative bacilli relative to patients without Proteobacteria domination in intestinal microbiota (Taur et al., Clin. Infect. Dis. 2012;55(7):905-914).

### SUMMARY OF THE APPLICATION

Provided herein is a method for determining the relative abundance of total bacterial in a sample. In one embodiment, the method includes (A) providing a fecal sample from a subject before the subject receives chemotherapy, (B) determining the relative abundance of total bacteria in the fecal sample, (C) detecting in the fecal sample bacteria that correlate with developing a bloodstream infection after chemotherapy and calculating the relative abundances of bacteria that correlate with developing a bloodstream infection after chemotherapy, and (D) detecting in the fecal sample bacteria that correlate with not developing a bloodstream infection after chemotherapy and calculating the relative abundances of bacteria that correlate with not developing a bloodstream infection after chemotherapy.

In one embodiment, determining the relative abundances in the fecal sample includes performing a quantitative polymerase chain reaction, such as by performing a high-throughput DNA sequencing. The determining can include analysis of a 16S rRNA variable region of the bacteria in the fecal sample, wherein the 16S rRNA variable region includes V1, V2, V3, V4, V5, V6, V7, V8, or V9, or a combination thereof. In one embodiment, the detecting of (C), (D), or both (C) and (D) includes analysis of the 16S rRNA region of the bacteria in the fecal sample. In one embodiment, the detecting of (C) includes an assay that detects a member of family Erysipelotrichaceae, a member of genus *Lactobacillus,* a member of genus *Eggerthella*, a member of genus *Veillonella*, or a combination thereof. The detecting of (D) includes an assay that detects a member of the order RF39 in the class Mollicutes, a member of the order Clostridiales, a member of family Barnesiellaceae, a member of family Coriobacteriaceae, a member of family Rikenellaceae, a member of genus *Butyricimonas,* a member of genus *Faecalibacterium,* such as *Faecalibacterium prausnitzii,* a member of genus *Oscillospira,* a member of family Christensenellaceae such as genus *Christensenella,* a member of genus *Dehalobacterium,* a member of genus *Desulfovibrio,* a member of genus *Sutterella,* a member of genus *Methanobrevibacter,* a member of genus Oxalobacter, or a combination thereof, wherein reference to a taxonomic group above species level excludes all lower taxonomic members of that group that are otherwise specifically identified.

The method can further include (E) generating a bloodstream infection risk index score, and (F) determining whether the subject has a risk of bloodstream infection in accordance with the result of (E). In one embodiment, a subject with a bloodstream risk index score that is greater than a threshold, such as from -1 to 1, is at higher risk of a blood stream infection after undergoing chemotherapy. In one embodiment, the chemotherapy is done to prepare the subject for a hematopoietic stem cell transplantation, and in another embodiment, the subject undergoes a hematopoietic stem cell transplantation after the chemotherapy. In one embodiment, the chemotherapy includes a non-myeloablative regimen or a myeloablative regimen.

In one aspect of the disclosure, the subject is at higher risk of a bloodstream infection after undergoing chemotherapy, and the method further includes administering to the subject a composition that includes at least one isolated bacteria. In one aspect, the at least one isolated bacteria administered is a member of the order RF39 in the class Mollicutes, a member of the order Clostridiales, a member of family Barnesiellaceae, a member of family Coriobacteriaceae, a member of family Rikenellaceae, a member of genus *Butyricimonas,* a member of genus *Faecalibacterium,* such as *Faecalibacterium prausnitzii,* a member of genus *Oscillospira,* a member of family Christensellaceae such as genus *Christensella,* a member of genus *Dehalobacterium,* a member of genus *Desulfovibrio,* a member of genus *Sutterella,* a member of genus *Methanobrevibacter,* a member of genus *Oxalobacter,* or a combination thereof. In one aspect, the administering includes administering at least 1x10³ of each isolated bacteria. The administering can occur at least 1 day before the subject undergoes the chemotherapy, while the subject undergoes the chemotherapy, after the subject undergoes the chemotherapy, or a combination thereof.

Also disclosedherein is a composition. In one aspect, the composition includes a mixture of isolated bacteria and a pharmaceutically acceptable carrier. The isolated bacteria are at least two selected from members of the order RF39 in the class Mollicutes, members of the order Clostridiales, members of family Barnesiellaceae, members of family Coriobacteriaceae, members of family Rikenellaceae, members of genus *Butyricimonas,* members of genus *Faecalibacterium,* such as *Faecalibacterium prausnitzii,* members of genus *Oscillospira,* members of family Christensellaceae such as genus *Christensella,* members of genus *Dehalobacterium,* members of genus *Desulfovibrio,* members of genus *Sutterella,* members of genus *Methanobrevibacter,* and members of genus *Oxalobacter.* In one aspect, the composition includes at least 1x10³ of each isolated bacteria. In one aspect, the composition can include at least three isolated bacteria, at least four isolated bacteria, at least five isolated bacteria, at least six isolated bacteria, at least seven isolated bacteria, at least eight isolated bacteria, at least nine isolated bacteria, at least ten isolated bacteria, at least eleven isolated bacteria, at least twelve isolated bacteria, or at least thirteen isolated bacteria.

Further disclosedherein is a method for preventing a bloodstream infection in a subject. In one aspect, the method includes administering to a subject in need thereof a composition that includes an effective amount of at least one isolated bacteria under conditions suitable for the isolated bacteria to populate the subject's gastrointestinal tract, wherein the risk that the subject will have a bloodstream infection is reduced. In oneaspect, the isolated bacteria is selected from members of the order RF39 in the class Mollicutes, members of the order Clostridiales, members of family Barnesiellaceae, members of family Coriobacteriaceae, members of family Rikenellaceae, members of genus *Butyricimonas,* members of genus *Faecalibacterium,* such as *Faecalibacterium prausnitzii,* members of genus *Oscillospira,* members of family Christensenella such as genus *Christensenella,* members of genus *Dehalobacterium,* members of genus *Desulfovibrio,* members of genus *Sutterella,* members of genus *Methanobrevibacter,* and members of genus *Oxalobacter.* In one aspect, the populations of at least two isolated bacteria, at least three isolated bacteria, at least four isolated bacteria, at least five isolated bacteria, at least six isolated bacteria, at least seven isolated bacteria, at least eight isolated bacteria, at least nine isolated bacteria, at least ten isolated bacteria, at least eleven isolated bacteria, at least twelve isolated bacteria, or at least thirteen isolated bacteria are administered. In one aspect, the subject is expected to receive chemotherapy. In one aspect, the chemotherapy is administered before the subject undergoes a hematopoietic stem cell transplant. In one aspect, a subject with a bloodstream risk index score that is greater than a threshold, such as from -1 to 1, is at higher risk of a blood stream infection after undergoing chemotherapy. The administering can include more than one administration, and the administration can be rectal, intubation through nose or mouth, oral, or a combination thereof. At least 1x10³ of each isolated bacteria can be administered. In one aspect, the administering occurs at least 2 days before the subject undergoes the chemotherapy. In one aspect, the isolated bacteria populate the subject's gastrointestinal tract for at least 1 day after the administration.

Also disclosed is a method for modifying the microbial population of a subject's gastrointestinal tract. In one aspect the method includes administering to a subject a composition that includes an effective amount of at least one isolated bacteria under conditions suitable for the isolated bacteria to populate the subject's gastrointestinal tract. The isolated bacteria can be selected from members of the order RF39 in the class Mollicutes, members of the order Clostridiales, members of family Barnesiellaceae, members of family Coriobacteriaceae, members of family Rikenellaceae, members of genus *Butyricimonas,* members of genus *Faecalibacterium,* such as *Faecalibacterium prausnitzii,* members of genus *Oscillospira,* members of family Christensenellaceae such as genus *Christensenella,* members of genus *Dehalobacterium,* members of genus *Desulfovibrio,* members of genus *Sutterella,* members of genus *Methanobrevibacter,* and members of genus *Oxalobacter.*

Further disclosed is a method for increasing the amount of bacteria in the gastrointestinal tract of a subject. In one aspect, the method includes administering to a subject a composition that includes an effective amount of at least one isolated bacteria under conditions suitable for the isolated bacteria to populate the subject's gastrointestinal tract, wherein the amount of the at least one isolated bacteria in the subject's gastrointestinal tract is increased compared to the amount of the at least one isolated bacteria before the administering. The isolated bacteria is selected from members of the order RF39 in the class Mollicutes, members of the order Clostridiales, members of family Barnesiellaceae, members of family Coriobacteriaceae, members of family Rikenellaceae, members of genus *Butyricimonas,* members of genus *Faecalibacterium,* such as *Faecalibacterium prausnitzii,* members of genus *Oscillospira,* members of family Christensenellaceae such as genus *Christensenella*, members of genus *Dehalobacterium,* members of genus *Desulfovibrio,* members of genus *Sutterella,* members of genus *Methanobrevibacter,* and members of genus *Oxalobacter.*

As used herein, the terms "subject" and "patient" are used interchangeably.

The term "and/or" means one or all of the listed elements or a combination of any two or more of the listed elements.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

It is understood that wherever embodiments are described herein with the language "include," "includes," or "including," and the like, otherwise analogous embodiments described in terms of "consisting of' and/or "consisting essentially of' are also provided.

Unless otherwise specified, "a," "an," "the," and "at least one" are used interchangeably and mean one or more than one.

Also herein, the recitations of numerical ranges by endpoints include all numbers subsumed within that range (e.g., 1 to 5 includes 1, 1.5, 2, 2.75, 3, 3.80, 4, 5, etc.).

For any method disclosed herein that includes discrete steps, the steps may be conducted in any feasible order. And, as appropriate, any combination of two or more steps may be conducted simultaneously.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### BRIEF DESCRIPTION OF THE FIGURES

Figure 1. Principal Coordinate Analysis (PCoA) of Unweighted UniFrac distance metric of bacterial communities from fecal samples from samples collected prior to treatment in patients who developed subsequent BSI (squares, n = 11) and in patients who did not develop subsequent BSI (circles, n = 17). 3,041 16S rRNA gene sequences were randomly selected from each sample.
Figure 2. Alpha-diversity indices m samples collected (PCoR) to treatment in patients who developed subsequent BSI (lower line in each graph, n = 11) versus samples collected prior to treatment in patients who did not develop subsequent BSI (upper line in each graph, n = 17). 3,041 16S rRNA gene sequences were randomly selected from each sample. A) Chao1 index, Monte Carlo permuted t-test: p = 0.002. B) Observed species, Monte Carlo permuted t-test: 0.001. C) Phylogenie distance whole tree, Monte Carlo permuted t-test: p = 0.001 D) Shannon index, Monte Carlo permuted t-test: p = 0.004.
Figure 3. Taxonomy bar charts of samples collected prior to chemotherapy in patients who developed subsequent BSI and in patients who did not develop subsequent BSI at family level.
Figure 4. Relative abundance of the most significant taxa in samples collected prior to treatment in patients who developed subsequent BSI (n = 11) and patients who did not develop BSI (n = 17). Mann-Whitney test: *: p < 0.05; **: p < 0.01 and ***: p < 0.001. Boxplots denote top quartile, median and bottom quartile.
Figure 5. ROC curve analysis of the most distinctive taxa in fecal samples collected prior to treatment following 10-fold cross-validation. The 10 ROC curves are in the dotted lines and the mean ROC curve is in solid black.
Figure 6. BSI Risk Index based on the differentiated taxa (n = 28). Mann---Whitney test: ***: p < 0.001. Boxplots denote top quartile, median and bottom quartile.
Figure 7. ROC curve analysis of the BSI Risk Index in fecal samples collected prior to treatment following 10-fold cross-validation. The 10 ROC curves are in the dotted line and the mean ROC curve is in solid black.
Figure 8. A) Most significantly altered metabolic pathways (L2 and L3 KEGG) in samples collected prior to treatment between patients who developed subsequent BSI (n = 11) and patients who did not develop BSI (n = 17). Mann-Whitney test: *: P < 0.05. Boxplots denote top quartile, median and bottom quartile. B) Linear Discriminant Analysis scores of differentially abundant microbial genes in gut microbiomes associated with or without subsequent BSI in fecal samples collected prior chemotherapy.
Figure 9. Presence of BSI-pathogens in fecal microbiomes of patients who did and did not develop BSI. The green dot represent the patient who developed a BSI corresponding to the plotted pathogen and the red dots represent the other patients (n = 27).
Figure 10. BSI Risk Index based on differentiated taxa in a previously published dataset (Taur et al., Clin. Infect. Dis. 2012;55(7):905-914). Mann-Whitney test: ns: non significant. Boxplots denote top quartile, median and bottom quartile.

### DETAILED DESCRIPTION OF ILLUSTRATIVE EMBODIMENTS

Subjects often undergo hematopoietic stem cell transplantation for treatment of hematological malignancy. However, bloodstream infection (BSI) is a common life-threatening complication during the early transplant period. There is a need for methods of identifying subjects who have an increased risk of developing a bloodstream infection such as after hematopoietic stem cell transplantation, after other transplantations that include a preliminary chemotherapy step, during chemotherapy and/or after chemotherapy received, for instance, in therapy for a cancer. Provided and disclosed herein are methods and kits for determining whether a subject has an increased risk for developing a bloodstream infection. Also disclosed are methods for treating a subject to reduce the risk of developing a bloodstream infection such as after hematopoietic stem cell transplantation, after other transplantations that include a preliminary chemotherapy step, during chemotherapy and/or after chemotherapy received, for instance, in therapy for a cancer. The treatment may occur before and/or during and/or after the chemotherapy.

In one embodiment, a method described herein includes determining risk of having a bloodstream infection. A fecal sample is obtained from a patient and is processed to measure the relative or fractional abundances of microbes in the sample. As used herein, "relative abundance" refers to the commonality or rarity of a microbe relative to other microbes in a fecal sample. For example, the relative abundance can be determined by generally measuring the presence of a particular microbe compared to the total presence of microbes in a sample. As used herein, "microbe" and "bacteria" are used interchangeably, and refer to prokaryotic cells that are members of the domain Bacteria and prokaryotic cells that are members of the domain Archaea. The patient is an individual who is expected to undergo chemotherapy. Examples of chemotherapies include those with doses high enough to prepare an individual for a transplant, such as hematopoietic stem cell transplantation (for instance, non-myeloablative, reduced intensity, and myeloablative conditioning regimens). Other examples of chemotherapies include those with doses generally used when the individual is being treated for solid tumors or blood cancers. The methods described herein are not limited by the type of compounds delivered to the individual for the chemotherapy. The patient may be any age, and may be any sex.

The fecal sample for determining risk may be obtained before chemotherapy begins. For instance, the sample may be obtained up to 31 days or fewer (31, 30, 29, 28, 27, 26, 25, 24, 23, 22, 21, 20, 19, 18, 17, 16, 15, 14, 13, 12, 11, 10, 9, 8, 7, 6, 5, 4, 3, 2, or 1) before chemotherapy begins, or it may be obtained the day chemotherapy begins.

The fecal sample is processed for evaluation of the bacterial genomic DNA present. Methods for processing a fecal sample to obtain bacterial genomic DNA that can be analyzed as described herein are known in the art and are routine.

Analysis of the bacterial genomic DNA is conducted to determine the relative or fractional abundances of different bacterial families, genera, or/and species in the sample. Any method for analysis of the bacterial genomic DNA to determine relative or fractional abundances may be used. In one embodiment, the relative bacterial load of different species in a fecal sample is determined. As used herein, "bacterial load" and "total bacterial load" are used interchangeably and refer to number of cells per unit of mass of feces by dry weight; "relative bacterial load" refers to the fraction of cells composed of a particular species, genus, family, or other taxonomic level of taxon in the bacterial community. In one embodiment, the total bacterial load per unit of fecal material by dry weight is measured. The total bacterial load per unit of fecal material can be measured using routine methods, and in one embodiment it is measured using quantitative PCR (qPCR) and universal DNA primers for the 16S ribosomal RNA (rRNA) gene. The quantity being measured would be the total number of copies of 16S rRNA genes per gram of fecal material by dry weight. Another common approach for determining the relative or fractional abundances of bacteria in the sample uses high-throughput DNA sequencing. Measurement of the total bacterial load is not required with high-throughput DNA sequencing because it already produces relative abundance measurements.

Analysis of the bacterial genomic DNA also includes measuring for two types of microbes: those correlating with developing a bloodstream infection after chemotherapy and those correlating with not developing a bloodstream infection after chemotherapy. Any method for determining the relative quantity of specific microbes present in a sample may be used. In one embodiment, strain identification is accomplished using nucleic acid based methods. Many methods based on molecular analyses are known in the art are routine (see, for instance, Li et al., 2009, FEMS Microbiol. Rev., 33:892916). In one embodiment, 16S rRNA gene identification is used. 16S rRNA gene identification is routinely used for identifying genus and species of many bacteria. These genes are highly conserved, but include specific hypervariable regions that contain sufficient nucleotide diversity to differentiate genera and species of most bacteria. Hypervariable regions of 16S rRNA that may be used include a V1, V2, V3, V4, V5, V6, V7, V8, or V9 region, or any combination thereof. Methods for using 16S rRNA analysis may include qPCR with primers specific to the species under consideration to determine the number of copies of 16S rRNA genes from a given species per gram of fecal material, and then normalizing these total abundance measurements by the total bacterial load obtained using universal primers and qPCR. The other common approach is to use universal primers to amplify a particular variable region of the 16S rRNA, and then subjecting the amplicons to high-throughput DNA sequencing, for example on the Illumina MiSeq instrument. Sources are readily available for use by the skilled person to determine if an amplification product identifies a specific genus or species, and include the Greengenes database available through the world wide web (http://greengenes.lbl.gov).

Bacteria whose presence correlates with developing a bloodstream infection after chemotherapy include, but are not limited to, members of the family Erysipelotrichaceae, members of the genus *Lactobacillus,* members of the genus *Eggerthella*, and members of the genus *Veillonella.*

Bacteria whose presence correlates with not developing a bloodstream infection after chemotherapy include, but are not limited to, members of the order RF39 in the class Mollicutes, members of the order Clostridiales, members of the family Barnesiellaceae, members of the family Coriobacteriaceae, members of the family Rikenellaceae, members of the genus *Butyricimonas,* members of the genus *Faecalibacterium,* members of the genus *Oscillospira,* members of the family Christensenellaceae, members of the genus *Christensenella,* members of the genus *Dehalobacterium,* members of the genus *Desulfovibrio,* members of the genus *Sutterella,* members of the genus *Methanobrevibacter,* and members of the genus *Oxalobacter.*

The method further includes generating a bloodstream infection risk index. Calculating this index includes determining the cumulative percent of each of the two groups of bacteria described herein, that is, the group of bacteria that correlate with developing a bloodstream infection after chemotherapy, and the group of bacteria that correlate with not developing a bloodstream infection after chemotherapy. For each sample collected, the relative abundance of each microbe is obtained and the dataset is normalized using arcsine of the square root. Then, in each sample the sum of bacteria that correlate with not developing a bloodstream infection after chemotherapy is calculated (unclassified members of RF39, unclassified members of Clostridiales, Barnesiellaceae, Coriobacteriaceae, Rikenellaceae, *Butyricimonas, Faecalibacterium, Oscillospira,* Christensenellaceae, *Christensenella, Dehalobacterium, Desulfovibrio, Sutterella, Methanobrevibacter,* and *Oxalobacter*). Then in each sample, the sum of the bacteria that correlate with developing a bloodstream infection is calculated (Erysipelotrichaceae, *Lactobacillus, Eggerthella*, *Veillonella*). In each case a taxonomic group above species level excludes all lower taxonomic members of that group that are specifically identified in another taxonomic group. For example, the calculation of relative abundance of Clostridiales excludes the family Christensenellaceae. Then, the BSI risk index is produced as the weighted or unweighted sum of the undesirable microbes (those correlating with developing a BSI) minus the weighted or unweighted sum of the desirable microbes (those correlating with not developing a BSI) in each sample. In one embodiment the weighted sum is used, and in another embodiment the unweighted sum is used.

Once the bloodstream infection risk index is calculated it can be used to determine whether the patient is at risk. A patient with a BSI risk index no greater than a given threshold is considered to have a lower risk of BSI after undergoing chemotherapy whereas a patient with a BSI risk index greater than that same threshold is considered at higher risk to develop a subsequent BSI during chemotherapy or after undergoing chemotherapy, such as chemotherapy for a hematopoietic stem cell transplantation (HSCT) procedure. The threshold may be any real-valued number from -1 to 1. For instance, the threshold may be -1, -0.9, -0.8, -0.7, -0.6, -0.5, - 0.4, -0.3, -0.2. -0.1, 0, 0.1, 0.2, 0.3, 0.4, 0.5, 0.6, 0.7, 0.8, 0.9, or 1. In one embodiment, the threshold may be a one hundredth, for instance, -0.02. In one embodiment, a model using leave-one-out cross validation is used to determine the threshold.

### Compositions

Also disclosed herein are compositions of bacteria. Cells in the composition may be vegetative cells, spores, or a combination thereof. The bacteria present in a composition may include one or more of the bacteria that are significantly differentiated between the patients that did and did not develop a BSI.

The bacteria present in a composition may include one or more members of the family Barnesiellaceae.

The bacteria present in a composition may include one or more members of the family Coriobacteriaceae.

The bacteria present in a composition may include one or more members of the family Rikenellaceae.

The bacteria present in a composition may include one or more members of the genus *Butyricimonas.*

The bacteria present in a composition may include one or more members of the genus *Faecalibacterium,* such as *Faecalibacterium prausnitzii.*

The bacteria present in a composition may include one or more members of the genus *Oscillospira.*

The bacteria present in a composition may include one or more members of the family Christensenellaceae.

The bacteria present in a composition may include one or more members of the genus *Christensenella.*

The bacteria present in a composition may include one or more members of the genus *Dehalobacterium.*

The bacteria present in a composition may include one or more members of the genus *Desulfovibrio.*

The bacteria present in a composition may include one or more members of the genus *Sutterella.*

The bacteria present in a composition may include one or more members of the genus *Methanobrevibacter.*

The bacteria present in a composition may include one or more members of the genus *Oxalobacter.*

The bacteria present in a composition may include one or more members of the order RF39 in the class Mollicutes.

The bacteria present in a composition may include one or more members of the order Clostridiales.

The bacteria present in a composition may include one or more members of other bacterial families, genera, or species in addition to those listed above.

A composition may include at least 1, at least 2, at least 3, at least 4, at least 5, at least 6, at least 7, at least 8, at least 9, at least 10, at least 11, at least 12, at least 13, at least 14, or at least 15 isolated bacteria. For instance, when a composition includes at least 3 isolated bacteria the composition may, in one aspect, include a member of the genus *Butyricimonas,* a member of the genus *Faecalibacterium,* and a member of the genus *Oscillospira,* or 2 members of the genus *Butyricimonas,* and a member of the genus *Faecalibacterium,* or any other combination of the bacteria described herein that are significantly differentiated between the patients that did and did not develop a BSI. In one aspect, a composition has at least 2 isolated bacteria, where the 2 bacteria are distinct strains of the same genus.

The numbers of each type of isolated bacteria in a composition may vary significantly depending upon the ultimate use of the composition. In those aspects where a composition is used for administration to a patient, a composition may include at least 1x10³, at least 1x10⁴, at least 1x10⁵, at least 1x10⁶, at least 1x10⁷, at least 1x10⁸, at least 1x10⁹, at least 1x10¹⁰, at least 1x10¹¹, or at least 1x10¹² bacterial cells. These numbers do not refer to the total number of isolated bacteria present, but the amount of each type of isolated bacteria. For instance, in one aspect a composition contains three isolated bacteria, and at least 1x10³ of each isolated bacteria is present in the composition.

The compositions disclosed herein may be included in a diversity of pharmaceutically acceptable formulations. In one aspect, a formulation may be a fluid composition. Fluid compositions include, but are not limited to, solutions, suspensions, dispersions, and the like. In one aspect, a formulation may be a solid composition. Solid compositions include, but are not limited to, powder, granule, compressed tablet, pill, capsule, chewing gum, wafer, and the like. Those formulations may include a pharmaceutically acceptable carrier to render the composition appropriate for administration to a subject. As used herein "pharmaceutically acceptable carrier" includes pharmacologically inactive compounds compatible with pharmaceutical administration. The compositions of the present disclosure may be formulated to be compatible with its intended route of administration. A composition of the present disclosure may be administered by any method suitable for depositing in the gastrointestinal tract of a subject. Examples of routes of administration include rectal administration (e.g., by suppository, enema, upper endoscopy, upper push enteroscopy, or colonoscopy), intubation through the nose or the mouth (e.g., by nasogastric tube, nasoenteric tube, or nasal jejunal tube), or oral administration (e.g., by a solid such as a pill, tablet, or capsule, or by liquid).

In one aspect, a composition may include one or more prebiotics, meaning compounds that are nutritious to or otherwise aid in growth of the bacteria being administered to a subject.

For therapeutic use in a method of the present disclosure, a composition may be conveniently administered in a form containing one or more pharmaceutically acceptable carriers. Suitable carriers are well known in the art and vary with the desired form and mode of administration of the composition. For example, they may include diluents or excipients such as fillers, binders, wetting agents, disintegrators, surface-active agents, glidants, lubricants, and the like. Typically, the carrier may be a solid (including powder), liquid, or combinations thereof. Each carrier is preferably "acceptable" in the sense of being compatible with the other ingredients in the composition and not injurious to the subject. The carrier is preferably biologically acceptable and inert, i.e., it permits the composition to maintain viability of the biological material until delivered to the appropriate site.

Oral compositions may include an inert diluent or an edible carrier. For the purpose of oral therapeutic administration, the bacteria can be incorporated with excipients and used in the form of tablets, troches, or capsules, e.g., gelatin capsules. Oral compositions can also be prepared by combining a composition of the present disclosure with a food. In one aspect a food used for administration is chilled, for instance, ice cream. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

A composition can also be prepared in the form of suppositories (e.g., with conventional suppository bases such as cocoa butter and other glycerides) or retention enemas for rectal delivery.

A composition may be prepared with carriers that will protect the bacteria against rapid elimination from the body, such as a controlled release formulation. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially. Liposomal suspensions can also be used as pharmaceutically acceptable carriers. These can be prepared according to methods known to those skilled in the art.

In one aspect, a composition may be encapsulated. For instance, when the composition is to be administered orally, the dosage form is formulated to protect the composition from conditions in some parts of the gastrointestinal tract, e.g., high acidity and digestive enzymes present in the stomach and/or parts of the intestine. The encapsulation of compositions for therapeutic use is routine in the art. Encapsulation may include hard-shelled capsules, which may be used for dry, powdered ingredients or soft-shelled capsules. Capsules may be made from aqueous solutions of gelling agents such as animal protein (e.g., gelatin), plant polysaccharides or derivatives like carrageenans and modified forms of starch and cellulose. Other ingredients may be added to a gelling agent solution such as plasticizers (e.g., glycerin and or sorbitol), coloring agents, preservatives, disintegrants, lubricants and surface treatment.

A composition may be prepared by routine culture of each of the bacteria that will be present in a composition. The bacteria used in the composition may be obtained from any source, including from a fecal sample or from strain collections. After appropriate numbers of each type of bacterium are available they may be combined into a composition, optionally with a pharmaceutically acceptable carrier. A composition may be used immediately, or stored for future use. Storage for future use may include freezing the cells, or further manipulating them into a suitable dosage form, such as a capsule.

### Methods of use

Also disclosed herein are methods for using the compositions described herein. In one aspect a method includes increasing the amount of certain bacteria in the gastrointestinal tract of a subject. In one aspect, a method includes treating certain conditions in a subject in need of treatment. Treatment of a condition can be prophylactic or, alternatively, can be initiated after the development of a condition. Treatment that is prophylactic, for instance, initiated before a subject manifests signs of a condition, is referred to herein as treatment of a subject that is "at risk" of developing a condition. An example of a subject that is at risk of developing a condition is a person having a risk factor. An example of a risk factor is the BSI risk index described herein. Other examples of a risk factor include undergoing chemotherapy before receiving a transplant, such as a HSCT. Treatment can be performed before, during, or after the occurrence of a condition described herein. Treatment initiated after the development of a condition may result in decreasing the severity of the signs of the condition, or completely removing the signs. Examples of conditions include a bloodstream infection, also referred to in the art as bacteremia and sepsis. Thus, in one aspect, a method includes preventing, e.g., reducing the risk, of a bloodstream infection in a patient. A subject may optionally be diagnosed as at risk, or not at risk, as described herein. Thus, in one aspect, a subject may be one that will undergo hematopoietic stem cell transplantation, one that will undergo some other transplantation that includes a preliminary chemotherapy step, one that is undergoing chemotherapy, or one that has undergone chemotherapy. In one aspect, the subject has a hematological malignancy, such as non-Hodgkin's lymphoma, myeloma, acute lymphocytic leukemia, or acute myelogenous leukemia. In one aspect, the subject is immunocompromised.

As used herein, the term "clinical sign," or simply "sign," refers to objective evidence of a condition present in a subject. As used herein, the term "symptom" refers to subjective evidence of a condition experienced by the patient and caused by condition. Symptoms and/or signs associated with conditions referred to herein and the evaluation of such signs are routine and known in the art.

In one aspect, a method includes transplanting a microbiota to a recipient. Such a transplantation results in modifying the microbial population of a subject's gastrointestinal tract. The method includes increasing the relative abundance in a recipient's gastrointestinal tract of the bacteria present in the composition administered to the subject. In this aspect, the phrase "relative abundance" refers to number in the recipient's gastrointestinal tract of the bacteria present in the composition administered to the subject compared to the number of all other bacteria in the recipient's gastrointestinal tract. Such a comparison can be expressed as a percent. In one aspect, the relative abundance in the recipient's gastrointestinal tract of the bacteria present in the composition administered to the subject after the administration may be increased by at least 0.01%, at least 0.05%, at least 0.1%, at least 0.5%, at least 1%, at least 5%, at least 10%, at least 20%, or at least 50%, compared to the recipient's gastrointestinal tract before the administration. These are relative percent increases, meaning that a change from 10% relative abundance to 15% relative abundance of a particular bacterial taxon would be considered a 50% increase and not a 5% increase. The change in the abundance may be determined at, for instance, 2 days, 3 days, 5 days, 7 days, 10 days, 14 days, or 25 days after the administration.

In one aspect, the microbiota already existing in the subject's gastrointestinal tract does not need to be cleared prior to administration of a composition described herein. In other ascpects clearance of the microbiota may be necessary. Whether clearance is necessary can be determined by an attending physician. Methods for clearance of existing microbiota are known and routine. In one example, clearance can be accomplished by administering a cocktail of antibiotics for one week until a day prior to transplant and/or a clearance preparation similar to those routinely used before colonoscopy. An example of a useful cocktail includes Metronidazole, Rifaximin, Vancomycin, and Neomycin.

### Kits

Also disclosed herein are kits. In one aspect, a kit is for detecting whether a patient is at risk of developing a bloodstream infection. The kit may include a fecal material collection apparatus of one or more containers and a solvent solution, and instructions for use. The kit may also include primers and/or probes suitable for use in determining the total bacterial load of a fecal sample, detecting in a fecal sample bacteria that correlate with developing a bloodstream infection after chemotherapy, detecting in a fecal sample bacteria that correlate with not developing a bloodstream infection after chemotherapy, or a combination thereof, and instructions for use. The primers may be in a suitable packaging material in an amount sufficient for at least one assay.

In one aspect, a kit is for treating a subject. The kit may include one or more containers with bacteria suitable for administering to a patient, and instructions for use. The bacteria may be vegetative cells, spores, or a combination thereof. The bacteria present in a kit may include one or more members of the family Barnesiellaceae; one or more members of the family Coriobacteriaceae; one or more members of the family Rikenellaceae; one or more members of the genus *Butyricimonas;* one or more members of the genus *Faecalibacterium,* such as *Faecalibacterium prausnitzii;* one or more members of the genus *Oscillospira*; one or more members of the family Christensenellaceae; one or more members of the genus *Christensenella;* one or more members of the genus *Dehalobacterium;* one or more members of the genus *Desulfovibrio;* one or more members of the genus *Sutterella;* one or more members of the genus *Methanobrevibacter;* one or more members of the genus *Oxalobacter;* one or more members of the order RF39 in the class Mollicutes; one or more members of the order Clostridiales, or a combination thereof, such as at least 2, at least 3, at least 4, at least 5, at least 6, at least 7, at least 8, at least 9, at least 10, at least 11, at least 12, at least 13, at least 14, or at least 15 isolated bacteria. The number of each isolated bacteria can be at least 1x10³, at least 1x10⁴, at least 1x10⁵, at least 1x10⁶, at least 1x10⁷, at least 1x10⁸, at least 1x10⁹, at least 1x10¹⁰, at least 1x10¹¹, or at least 1x10¹² bacterial cells. The bacteria present in a kit may include one or more members of other bacterial families, genera, or species in addition to those listed above.

As used herein, the phrase "packaging material" refers to one or more physical structures used to house the contents of the kit. The packaging material is constructed by known methods, preferably to provide a sterile, contaminant-free environment. The packaging material has a label which indicates that the disclosure may be used for its intended purpose. In addition, the packaging material contains instructions indicating how the materials within the kit are employed. As used herein, the term "package" refers to a solid matrix or material such as glass, plastic, paper, foil, and the like, capable of holding within fixed limits primers or bacteria. Thus, for example, a package can be a plastic vial used to contain milligram quantities of a primer. "Instructions for use" typically include a tangible expression describing the reagent concentration or at least one assay method parameter, such as the relative amounts of reagent and sample to be admixed, maintenance time periods for reagent/sample admixtures, temperature, buffer conditions, and the like.

### Example 1

### ABSTRACT

Hematopoietic stem cell transplantation (HSCT) is a curative treatment in patients with hematological malignancy. Bloodstream infection (BSI) is a common life-threatening complication during the early transplant period. To date, the impact of the pre-treatment intestinal microbiome on risk of BSI remains unclear. The objective of our work was to characterize the fecal microbiome collected prior to chemotherapy to identify taxonomic and functional microbiome features associated with the risk of BSI. Fecal samples were collected from 28 patients with non-Hodgkin's lymphoma prior to administration of chemotherapy and 16S rRNA genes were characterized using high-throughput DNA sequencing. Machine learning tools were applied to develop predictive biomarkers for BSI. We found that pre-treatment fecal samples of the patients who developed subsequent BSI exhibited a decreased overall diversity and a decreased abundance of taxa including Barnesiellaceae, Coriobacteriaceae, *Faecalibacterium, Christensenella, Dehalobacterium, Desulfovibrio* and *Sutterella.* We developed a BSI risk index capable of predicting BSI incidence with 86% accuracy based on the pre-treatment microbiome. Our analysis showed that significant differences in microbial community structure in advance of treatment precede BSI, and that analysis of the microbial community can predict BSI in new patients.

To date, the impact of the intestinal microbiome before treatment initiation on the risk of subsequent BSI remains poorly studied. Thus, the objective of our work was to study fecal samples collected prior to chemotherapy to identify features of the fecal microbiome associated with the risk of subsequent BSI.

### RESULTS

### Patient and fecal sample characteristics.

During the study period, 28 patients with non-Hodgkin's lymphoma undergoing HSCT were included. Clinical characteristics of the patients are listed in Table 1. Overall, 28 fecal samples collected prior to chemotherapy were collected. Of the fecal samples collected, a total of 280,416 high-quality 16S rRNA-encoding sequences were identified, representing 3,857 OTUs (operational taxonomic units). The mean number of sequences obtained per sample was 10,015 ± 4,2964. Importantly, since samples contained between 3,041 and 26,122 sequences, diversity analyses were rarefied at 3,041 sequences per sample to avoid bias.

**Table 1. Characteristics of the study population. BSI: Bloodstream infection**

| | n (%, 95%CI or median, 1^{st} and 3^{rd} quartile) |
|---|---|
| Age (years) | 55 [45-62] |
| Sex, male | 18 (64.3%, 95%CI: 44.1- 80.7%) |
| Body Mass Index | 24 [23-27] |
| Antibiotic prophylaxis | 24 (85.7%, 95%CI: 66.4-95.3%) |
| penicillin V | 14 (50.0%, 95%CI: 32.6-67.4%) |
| cotrimoxazole | 18 (64.3%, 95%CI: 44.1- 80.7%) |
| Previous history of chemotherapy | 27 (96.4%, 95%CI: 79.7-99.8%) |
| BSI | 11 (39% [24% to 58%]) |
| *Escherichia coli* | 4 (36.4% [15.0% to 64.8%]) |
| *Enterococcus* | 2 (18.2% [39.9% to 48.9%]) |
| other Gammaproteobacteria | 5 (45.5% [21.3% to 72.0%]) |

BSI was reported in 11 patients (39% [24% to 58%]), by a mean of 12 ± 1 days after sample collection. Two patients (18.2% [39.9% to 48.9%] developed *Enterococcus* BSI, 4 patients (36.4% [15.0% to 64.8%] developed *Escherichia coli* BSI and 5 (45.5% [21.3% to 72.0%] patients developed other Gammaproteobacteria BSI (Table 1).

### Decreased diversity in pre-chemotherapy fecal samples associated with subsequent BSI.

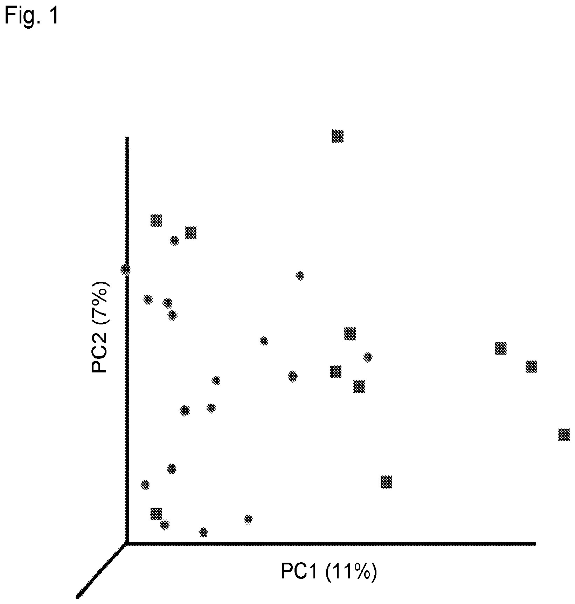

PCoA of fecal samples collected prior to treatment, based on 16S rRNA sequences of unweighted UniFrac distance metric showed differences between fecal samples of patients who did or did not develop BSI (Figure 1). Moreover, the ANOSIM method determined that fecal bacterial communities diverged significantly between samples of the patients who did or did not develop BSI (R = 0.30, p = 0.01).

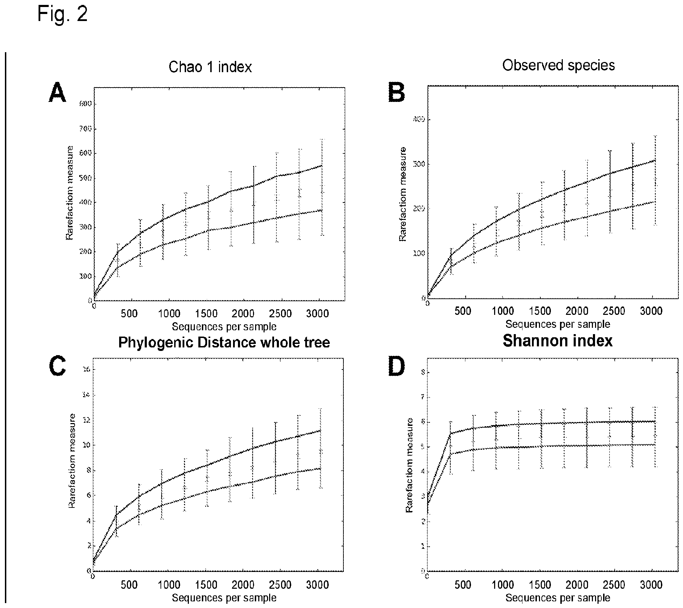

Alpha diversity in fecal samples from patients who developed BSI was significantly lower than alpha diversity from patients who did not develop subsequent BSI, with reduced evenness and reduced richness (Figure 2, Table 2).

**Table 2. Comparison of alpha diversity in patients who developed subsequent BSI and patients who did not develop BSI. Values are given as mean ± SD. BSI: Bloodstream infection, PD: Phylogenetic Distance whole tree.**

| | Chao1 | Shannon | PD | Observed species |
|---|---|---|---|---|
| BSI | 327 ± 94 | 5.1 ± 0.9 | 7.2 ± 1.2 | 188.8 ± 46 |
| No BSI | 495 ± 89.6 | 6 ± 0.7 | 10 ± 1.7 | 269.7 ± 49.4 |
| p value between BSI and no BSI | 0.002 | 0.004 | 0.001 | 0.001 |

Machine learning tools (Random Forest classifier) were also used to determine the robustness of clustering fecal samples from patients who did or did not develop BSI (14). The model classified unknown samples with a 0.15 ± 0.20 error rate, which is 2.6 times better than the baseline error rate for random guessing.

Thus, patients who developed subsequent BSI exhibited a specific pre-chemotherapy decreased diversity of fecal microbiota compared to patients who did not develop BSI. Furthermore, Random Forest, used as a machine learning strategy, determined that pre-treatment fecal microbiome was highly predictive of the occurrence of subsequent BSI.

### Taxa associated with the risk of subsequent BSI.

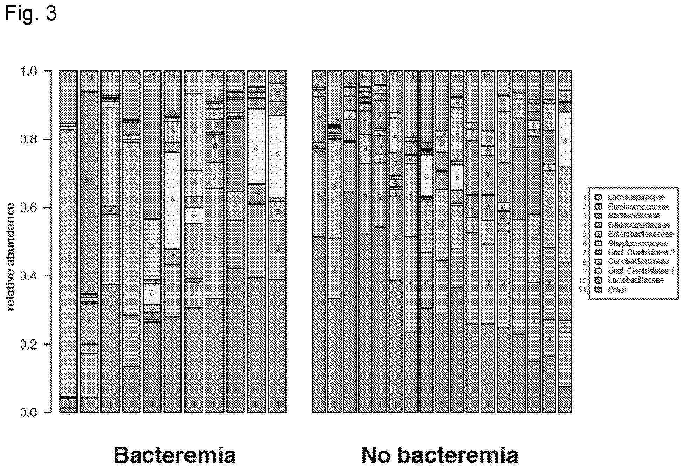

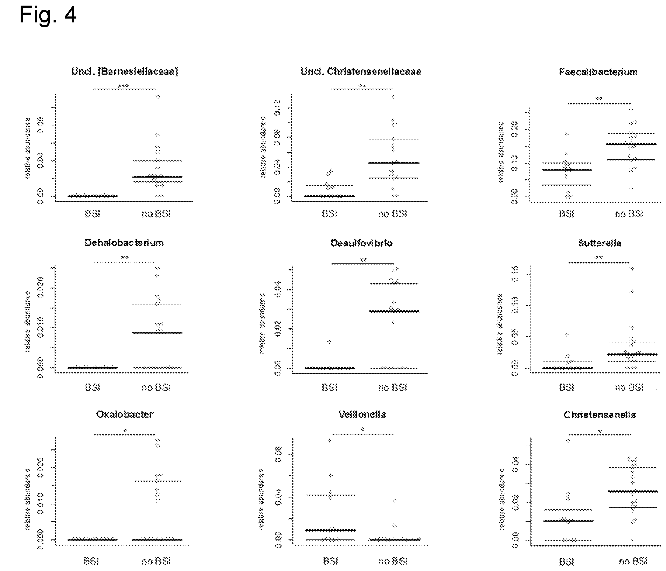

To discover which bacterial groups are driving the differences between patients who did and did not develop BSI, we identified taxa that reached statistically significant association with the risk of subsequent BSI using a linear model with false discovery rate correction (FDR) and a Random Forest classifier. Based on these tools, we identified a panel of 19 taxa that were highly differentiated between patients who did and did not develop BSI. Fecal samples collected prior to treatment from the patients who developed subsequent BSI exhibited significantly decreased abundance of members of Bacteroidetes (Barnesiellaceae, Rikenellaceae, *Butyricimonas*), Firmicutes (Christensenellaceae, *Faecalibacterium, Oscillospira, Christensenella*, *Dehalobacterium*), Proteobacteria (*Desulfovibrio, Sutterella, Oxalobacter*)*,* Actinobacteria (Coriobacteriaceae), the order RF39 in the class Mollicutes, and the order Clostridiales compared to patients who did not develop subsequent BSI. The patients who developed BSI exhibited significantly higher abundance of Erysipelotrichaceae, *Lactobacillus, Eggerthella,* and *Veillonella* in fecal samples collected prior to treatment compared to patients who did not develop subsequent BSI (Table 3, Table 4, Figure 3, Figure 4).

**Table 3. Families that differ between fecal samples collected prior to treatment in patients who did or did not develop BSI. Q values are p values adjusted with a false discovery rate correction**

| Feature | P value | Q value | Bacteremia mean | No bacteremia mean |
|---|---|---|---|---|
| k_Bacteria;p_Bacteroidetes;c_Bacteroidia;o_Bacteroidales;f_ [Barnesiellaceae] | 4.80123E-05 | 0.002160555 | 0 | 0.030608691 |
| k_Bacteria;p_Firmicutes;c_ Clostridia;o_Clostridiales;f_Christensenellaceae | 0.000976047 | 0.021961056 | 0.017263335 | 0.061397059 |
| k_Bacteria;p_Proteobacteria;c_Deltaproteobacteria;o_Desulfovibrionales;f_Desulfovibrionaceae | 0.002583052 | 0.038745776 | 0.009117493 | 0.035661785 |
| k_Bacteria;p_Firmicutes;c_ Clostridia;o_Clostridiales;f_ Dehalobacteriaceae | 0.005454802 | 0.061366525 | 0 | 0.008590271 |
| k_Bacteria;p_Proteobacteria;c_Betaproteobacteria;o_Burkholderiales;f_Alcaligenaceae | 0.008700052 | 0.077199038 | 0.00824569 | 0.0361082 |
| k_Bacteria;p_Tenericutes;c_Mollicutes;o_RF39;f_ | 0.010293205 | 0.077199038 | 0 | 0.033369899 |
| k_Bacteria;p_Proteobacteria;c_Betaproteobacteria;o_Burkholderiales;f_ Oxalobacteraceae | 0.015134529 | 0.085608235 | 0.003111599 | 0.011823771 |
| k_Bacteria;p_Bacteroidetes;c_ Bacteroidia;o_Bacteroidales;f_ [Odoribacteraceae] | 0.015219242 | 0.085608235 | 0.01364343 | 0.037690691 |
| k_Bacteria;p_Firmicutes;c_Bacilli;o_Lactobacillales;f_Lactobacillaceae | 0.036943059 | 0.157390604 | 0.111370127 | 0.017814454 |
| k_Bacteria;p_Firmicutes;c_ Clostridia;o_Clostridiales;f_ | 0.038473259 | 0.157390604 | 0.142158954 | 0.238485423 |
| k_Bacteria;p_Firmicutes;c_ Clostridia;o_Clostridiales;f_ Ruminococcaceae | 0.038473259 | 0.157390604 | 0.36528884 | 0.462636458 |
| k_Archaea;p_Euryarchaeota;c_Methanobacteria;o_Methanobacteriales;f_Methanobacteriaceae | 0.059093078 | 0.204885444 | 0.001120912 | 0.008159233 |
| k_Bacteria;p_Bacteroidetes;c_Bacteroidia;o_Bacteroidales;f_Rikenellaceae | 0.059189128 | 0.204885444 | 0.039500358 | 0.081755497 |
| k_Bacteria;p_Firmicutes;c_Bacilli;o_Lactobacillales;f_Enterococcaceae | 0.08160517 | 0.249196743 | 0.079175552 | 0.032833039 |
| k_Bacteria;p_Actinobacteria;c_Actinobacteria;o_Actinomycetales;f_Micrococcaceae | 0.083089608 | 0.249196743 | 0.025916499 | 0.01212222 |
| k_Bacteria;p_Bacteroidetes;c_ Bacteroidia;o_Bacteroidales;f_Bacteroidaceae | 0.090369145 | 0.249196743 | 0.19337139 | 0.276421604 |
| k_Bacteria;p_Bacteroidetes;c_Bacteroidia;o_Bacteroidales;f_Porphyromonadaceae | 0.094480305 | 0.249196743 | 0.062308631 | 0.098703364 |
| k_Bacteria;p_Firmicutes;c_Bacilli;o_Lactobacillales;f_ Streptococcaceae | 0.099678697 | 0.249196743 | 0.236983183 | 0.134896497 |

**Table 4. Genera that differ between fecal samples collected prior to treatment in patients who did or did not develop bloodstream infection (BSI). Q values are p values adjusted with a false discovery rate correction**

| Feature | P value | Q value | Bacteremia mean | Nobacteremia mean |
|---|---|---|---|---|
| k_Bacteria;p_Bacteroidetes;c_Bacteroidia;o_Bacteroidales;f_[Barnesiellaceae];g_ | 4.80123E-05 | 0.0035049 | 0 | 0.030608691 |
| k_Bacteria;p_Firmicutes;c_ Clostridia;o_Clostridiales;f_Christensenellaceae;g_ | 0.002715736 | 0.093925496 | 0.009486937 | 0.051637282 |
| k_Bacteria;p_Firmicutes;c_ Clostridia;o_Clostridiales;f_ Ruminococcaceae;g_Faecalibacterium | 0.004106589 | 0.093925496 | 0.075943204 | 0.157567367 |
| k_Bacteria;p_Firmicutes;c_Clostridia;o_Clostridiales;f_Dehalobacteriaceae;g_Dehalobacterium | 0.005454802 | 0.093925496 | 0 | 0.008110837 |
| k_Bacteria;p_Proteobacteria;c_Deltaproteobacteria;o_Desulfovibrionales;f_Desulfovibrionaceae;g_ Desulfovibrio | 0.006485006 | 0.093925496 | 0.001185782 | 0.022130464 |
| k_Bacteria;p_Proteobacteria;c_Betaproteobacteria;o_Burkholderiales;f_Alcaligenaceae;g_Sutterella | 0.008700052 | 0.093925496 | 0.00824569 | 0.0361082 |
| k_Bacteria;p_Proteobacteria;c_Betaproteobacteria;o_Burkholderiales;f_ Oxalobacteraceae;g_Oxalobacter | 0.010293205 | 0.093925496 | 0 | 0.008363649 |
| k_Bacteria;p_Tenericutes;c_Mollicutes;o_RF39;f_ ;g_ | 0.010293205 | 0.093925496 | 0 | 0.033369899 |
| k_Bacteria;p_Firmicutes;c_ Clostridia;o_Clostridiales;f_ Veillonellaceae;g_Veillonella | 0.014371074 | 0.116565377 | 0.023380631 | 0.002898584 |
| k_Bacteria;p_Firmicutes;c_Clostridia;o_Clostridiales;f_Christensenellaceae;g_Christensenella | 0.016143634 | 0.117848525 | 0.012657346 | 0.026329171 |
| k_Bacteria;p_Firmicutes;c_Clostridia;o_Clostridiales;f_Ruminococcaceae;g_Oscillospira | 0.018671821 | 0.118013149 | 0.039894856 | 0.072200504 |
| k_Bacteria;p_Bacteroidetes;c_Bacteroidia;o_Bacteroidales;f_ [Odoribacteraceae];g_ Butyricimonas | 0.019399422 | 0.118013149 | 0.005621577 | 0.018437991 |
| k_Bacteria;p_Firmicutes;c_Erysipelotrichi;o_Erysipelotrichales;f_Erysipelotrichaceae;g_ | 0.02394951 | 0.134485712 | 0.082986672 | 0.029542167 |
| k_Bacteria;p_Firmicutes;c_ Clostridia;o_Clostridiales;f_;g_ | 0.038473259 | 0.200610563 | 0.142158954 | 0.238485423 |
| k_Bacteria;p_Firmicutes;c_Bacilli;o_Lactobacillales;f_Lactobacillaceae;g_Lactobacillus | 0.047050679 | 0.227410861 | 0.102110686 | 0.015694449 |
| k_Bacteria;p_Actinobacteria;c_Coriobacteriia;o_Coriobacteriales;f_Coriobacteriaceae;g_ | 0.053740851 | 0.227410861 | 0.062307631 | 0.113406706 |
| k_Bacteria;p_Actinobacteria;c_Coriobacteriia;o_Coriobacteriales;f_Coriobacteriaceae;g_Eggerthella | 0.053830644 | 0.227410861 | 0.024139376 | 0.006111561 |
| k_Archaea;p_Euryarchaeota;c_Methanobacteria;o_Methanobacteriales;f_Methanobacteriaceae;g_Methanobrevibacter | 0.059093078 | 0.227410861 | 0.001120912 | 0.008159233 |
| k_Bacteria;p_Bacteroidetes;c_Bacteroidia;o_Bacteroidales;f_Rikenellaceae;g_ | 0.059189128 | 0.227410861 | 0.03945261 | 0.081711169 |

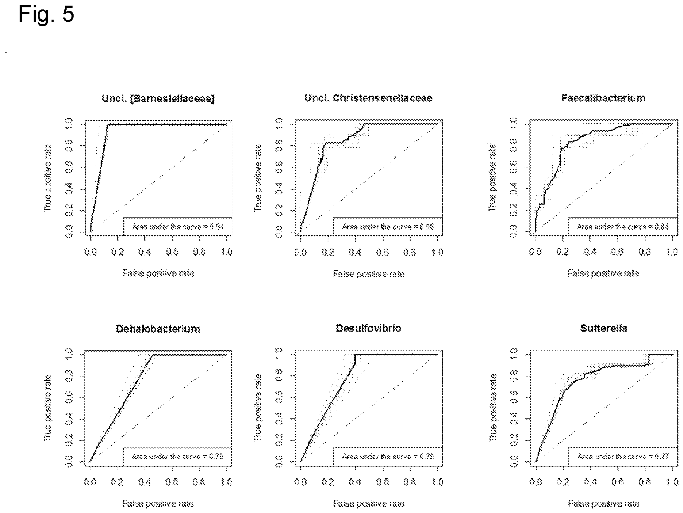

We tested the ability of this panel of microbes to discriminate between patients who did and did not develop subsequent BSI. Based on *ROC curve analysis,* we found that Barnesiellaceae yielded a ROC-plot AUC value of 0.94, *Christensenellaceae* yielded a ROC-plot AUC value of 0.86, *Faecalibacterium* yielded a ROC-plot AUC value of 0.84, *Desulfovibrio* yielded a ROC-plot AUC value of 0.79, *Dehalobacterium* yielded a ROC-plot AUC value of 0.78 and Sutterella yielded a ROC-plot AUC value of 0.77 (Figure 5).

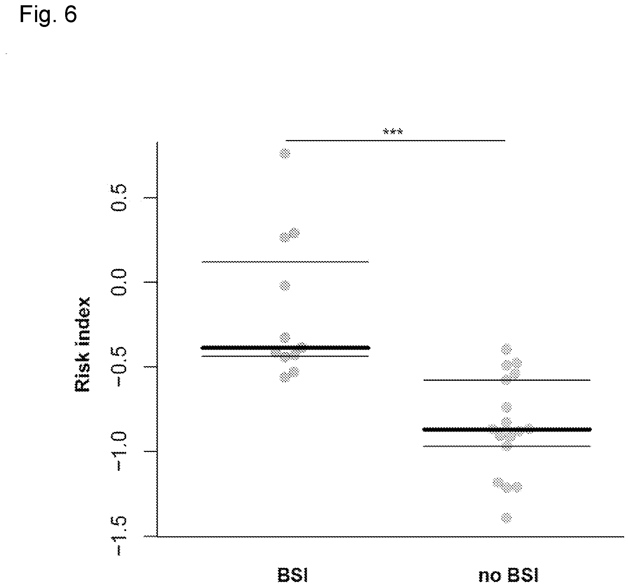

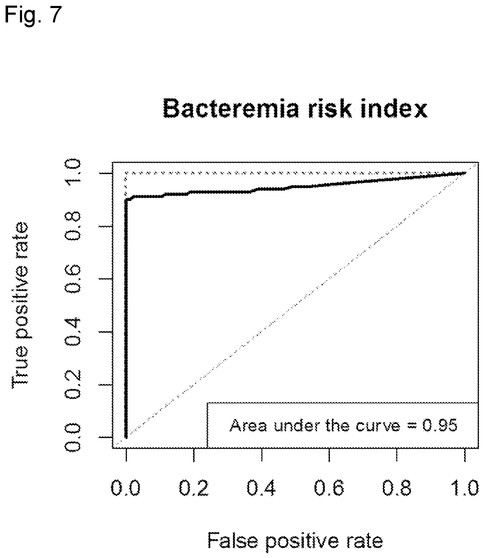

We then built a BSI risk index from this panel of microbes. As shown in Figure 6, our BSI Risk Index highly differentiated between patients who did and did not develop BSI. Median BSI Risk index was -0.39 (IQR= 0.56) in patients who develop subsequent bacteremia and median BSI risk index was -0.87 (IQR = 0.39) in patients who did not develop BSI (Mann-Whitney U test, p < 0.001). Moreover, *ROC curve analysis* showed that BSI risk index was a strong predictor of the onset of subsequent BSI, with an AUC of 0.95 (Figure 7).

We also determined a BSI risk index threshold that best predicts bacteremia with leave-one-out (LOO) cross-validation. Each held-out sample was treated as a new patient on whom we tested and subsequently refined the optimal BSI index cutoff to separate patients who developed BSI and patients who did not develop BSI. In this model, the median BSI risk index in patients who developed bacteremia was -0.33 (IQR = 0.26), and in patients that did not develop bacteremia it was -0.85 (IQR = 0.60). This procedure demonstrated that the accuracy of a cutoff of -0.44 to predict BSI in a new patient was 86% at a specificity of 95%.

Moreover, association between clinical data (age, sex, previous antibiotic treatment received, type of antibiotic treatment, time since the previously received antibiotics, previous chemotherapy received, time since the previously received chemotherapy) and BSI was tested using a multivariate logistic regression with a backward procedure. No significant association was found between all clinical data and BSI (Table 5). Furthermore, we evaluated the association between microbes and these clinical characteristics (Table 6). We found in our study that a previously received antibiotic treatment was associated with decreased Firmicutes (Gemellales, Clostridiaceae, Coriobacteriaceae) and that previously received chemotherapy was associated with a decrease in Firmicutes (Gemellales, Clostridiales, *Dehalobacterium, Faecalibacterium*) and Actinobacteria (Coriobacteriaceae, Propionibacteriaceae).

**Table 5. Association between clinical characteristics of the patients and the outcome (BSI) based on a logistic regression model with backward.**

| | Estimate | Std. Error | z value | Pr(>\|z\|) |
|---|---|---|---|---|
| (Intercept) | 23.50988 | 2399.54961 | 0.010 | 0.9922 |
| Antibiotic Oracilline | -1.48463 | 1.09895 | -1.351 | 0.1767 |
| Antibiotic Bactrim | 1.63217 | 1.03467 | 1.577 | 0.1147 |
| Chemotherapy previous use | -18.76785 | 2399.54562 | -0.008 | 0.9938 |
| Delay of the Chemotherapy | -3.88820 | 2.14295 | -1.814 | 0.0696 |
| Age of the patient | -0.07561 | 0.05462 | -1.384 | 0.1663 |

**Table 6. Association between clinical characteristics of the patients and taxonomy at genus level.**

| Feature | Taxon | pvalue | qvalue | coefficient |
|---|---|---|---|---|
| Antibiotherapy Oracilline | k_Bacteria;p_Firmicutes;c_Clostridia;o_ Clostridiales;f_Clostridiaceae;g_ | 0.00153925 | 0.104668975 | -0.030608421 |
| Covariate | Taxon | pvalue | qvalue | coefficient |
| Antibiotherapy Bactrim | k_Bacteria;p_Firmicutes;c_Clostridia;o_ Clostridiales;f_Clostridiaceae;g_ | 0.004393394 | 0.168218593 | -0.027548298 |
| Antibiotherapy Bactrim | k_Bacteria;p_Actinobacteria;c_ Coriobacteriia;o_Coriobacteriales;f_Coriobacteriaceae;Other | 0.004947606 | 0.168218593 | -0.008401964 |
| Antibiotherapy Bactrim | k_Bacteria;p_Firmicutes;c_Bacilli;o_Gemellales;Other;Other | 0.007681242 | 0.17410815 | -0.012430685 |
| Covariate | Taxon | pvalue | qvalue | coefficient |
| chemotherapy | k_Bacteria;p_Actinobacteria;c_Coriobacteriia;o_Coriobacteriales;f_Coriobacteriaceae;g_ | 0.000542616 | 0.019493257 | -0.320695974 |
| chemotherapy | k_Bacteria;p_Actinobacteria;c_Actinobacteria;o_Actinomycetales;f_Propionibacteriaceae;g_ | 0.000573331 | 0.019493257 | -0.052757802 |
| chemotherapy | k_Bacteria;p_Firmicutes;c_Bacilli;o_ Gemellales;Other;Other | 0.001132333 | 0.025666207 | -0.044092461 |
| chemotherapy | k_Bacteria;p_Firmicutes;c_Clostridia;o_ Clostridiales;Other;Other | 0.002902286 | 0.049338855 | -0.158556689 |
| chemotherapy | k_Bacteria;p_Proteobacteria;c_Deltaproteobacteria;o_Desulfovibrionales;f_Desulfovibrionaceae; g_ Desulfovibrio | 0.010167119 | 0.138272815 | -0.059347826 |
| chemotherapy | k_Bacteria;p_Firmicutes;c_Clostridia;o_ Clostridiales;f_ Dehalobacteriaceae;g_Dehalobacterium | 0.01766588 | 0.200213305 | -0.021055751 |
| chemotherapy | k_Bacteria;p_Actinobacteria;c_Coriobacteriia;o_Coriobacteriales;f_Coriobacteriaceae;Other | 0.021532797 | 0.209175744 | -0.019571738 |
| Covariate | Taxon | pvalue | qvalue | coefficient |
| delay chemotherapy > 5 months | k_Bacteria;p_Firmicutes;c_Bacilli;o_ Lactobacillales;f_Streptococcaceae;g_ Lactococcus | 0.000193445 | 0.013154254 | 0.046963203 |
| delay chemotherapy > 5 months | k_Bacteria;p_Firmicutes;c_Clostridia;o_Clostridiales;f_Lachnospiraceae;g_[Ruminococcus] | 0.003329648 | 0.113208045 | 0.144742429 |

Thus, prior to administration of high-dose chemotherapy conditioning the transplant, the fecal microbiome of patients that developed subsequent BSI exhibited a specific decreased overall diversity and a distinct bacterial composition compared to patients that did not develop BSI. The mean time in this study between this indicative taxonomic imbalance and BSI was 12 days.

### Differences in the microbiome functional repertoire in patients who developed subsequent BSI.

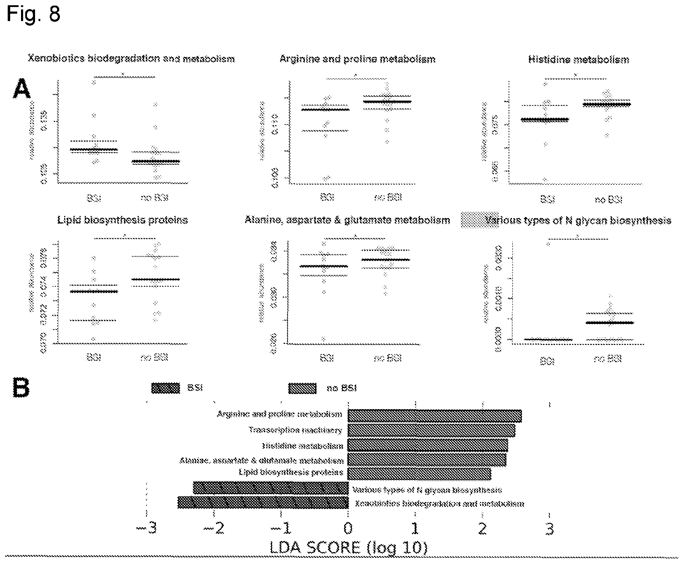

We predicted the functional composition of the fecal microbiome using PICRUSt. This algorithm estimates the functional potential of microbial communities given the current 16S rRNA gene survey and the set of currently sequenced reference genomes (15). We used LEfSe to identify significant differences in microbial genes (level 2 and level 3 KEGG Orthology groups) in the samples collected prior to treatment from patients who developed and did not develop subsequent BSI (16). The fecal microbiome of patients that developed subsequent bacteremia were enriched in functional categories associated with xenobiotics biodegradation and metabolism and depleted in categories associated with glycan biosynthesis, transcription machinery, histidine metabolism, arginine and proline metabolism, lipid biosynthesis proteins and alanine, aspartate and glutamate metabolism (Figure 8).

We found a loss of glycan biosynthesis in patients who developed subsequent BSI. Glycans are well-described factors of the intestinal microbiome homeostasis (17-20). Mice with induced glycan deficiency presented a rapid decrease in mucus layer thickness associated with increased bacterial translocation (21). Another study reported that altered Muc2 homeostasis, resulting from the absence of an enzyme implicated in the biosynthesis of glycans, was associated with an increase in bacterial translocation (22). Thus, loss of glycan biosynthesis categories increases the permeability of the intestinal barrier, thus favoring bacterial translocation (23, 24).

We also found an increase in xenobiotics biodegradation and metabolism in patients who developed subsequent bacteremia. This metabolic pathway, previously found decreased in Crohn's disease, has been reported to promote Enterobacteriaceae growth and virulence (25-27). Interestingly, this taxonomic family represents the most frequent pathogens isolated in cases of BSI in our population.

We also found that the intestinal microbiome in patients who developing a subsequent BSI was associated with a decrease of proline metabolism. This amino acid was previously associated with decreased mucin synthesis, impaired colonic protection and impaired intestinal mucosal barrier (28). We also showed a decreased in histidine metabolism in patients with subsequent BSI. Histidine was found decreased in HIV patients associated with intestinal inflammation, favoring bacterial translocation (29). Moreover, histidine supplementation protected the intestinal tissue and reduce bacterial translocation in Salmonella-challenged mouse enabling resistance to the oxidative stress during the acute inflammatory response (30). Furthermore, histidine has the ability to hydrogen peroxide- and TNF-α-induced IL-8 secretion, suggesting that histidine can suppress intestinal inflammation (31).

We also found that depleted arginine metabolism category was associated with subsequent bacteremia. In rats infected with *E. coli*, inoculated into the intestinal lumen, arginine supplementation reduced the level of bacterial translocation when compared with the control group (32). Moreover, several studies reported that arginine supplementation prevent damage to the intestinal mucosa and favor the intestinal barrier function and therefore preventing bacterial translocation (33-35).

Glutamate, found decreased in patients who developed a subsequent BSI, also protects the mucosal integrity and reduces bacterial translocation. In rats, glutamate supplementation reduced intestinal permeability and bacterial translocation caused by trauma and endotoxemia (36). Moreover, a randomized trial showed that glutamate supplementation reduced significantly the rate of sepsis in low-birth-weight infants (37). In the case of intestinal inflammation, the availability of glutamate is pivotal for the maintenance of an efficient barrier function and glutamine deprivation enables bacterial translocation (38).

Here we found that a specifically functional imbalance in fecal samples collected prior to treatment was associated with subsequent BSI. These alterations in the metabolic capacity were previously reported to compromise the intestinal epithelial barrier function, therefore enabling bacterial translocation.

### Comparison between BSI pathogen sequences and patient fecal sample sequences.

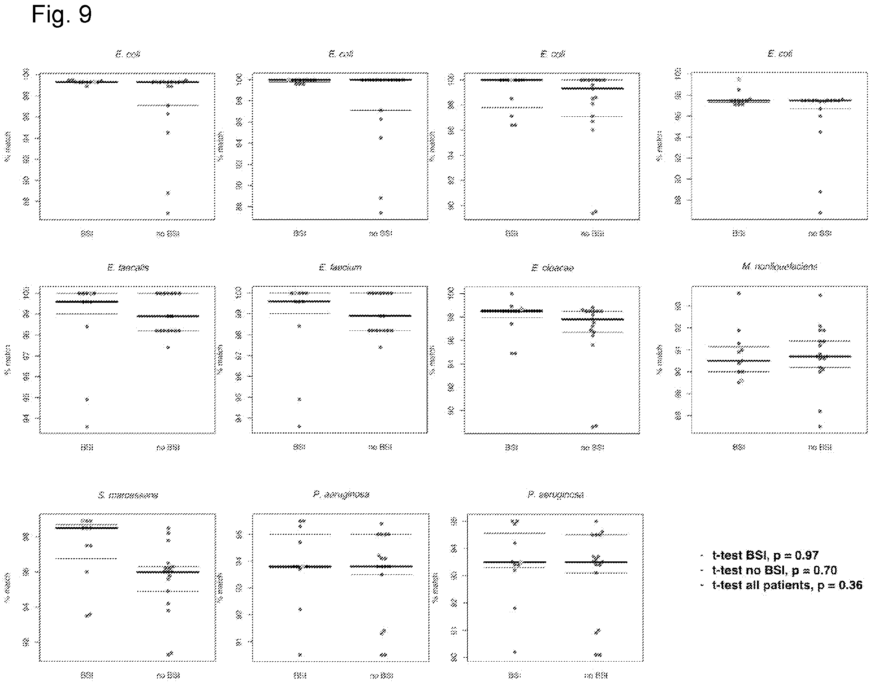

We compared the BSI pathogen sequence to each patient's fecal microbiome sequences using usearch and ublast command (39). We found that the 16S rRNAsequence of each BSI pathogen was very similar to at least one sequence in the feces of the patients, as well as in the feces of patients who did not develop BSI. Tests showed no statistical difference in the occurrence of BSI-pathogen sequences in feces of either group (p = 0.97, p = 0.70) (Figure 9). Based on these results, we propose that all the patients may have the pathogenic taxa in their gut but only a specific community-wide taxonomic and functional dysbiosis, as described above, will eventually lead to translocation and invasion of the host systemic organs.

### Bacteremia prediction in a previously published dataset.

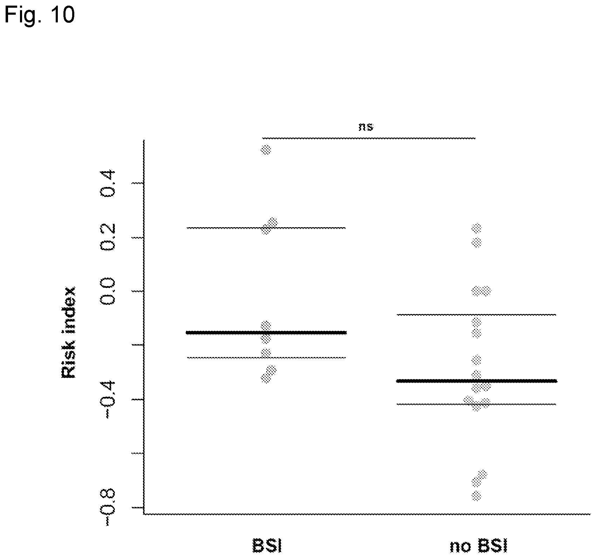

We then applied our BSI risk index in a previously published dataset (13). From this dataset, 24 patients had samples publicly available at http://www.ncbi.nlm.nih.gov/sra?term=SRP045811. To build the BSI risk index in this cohort, we included only fecal samples collected before the day of the transplant. We showed that patients who developed a subsequent BSI had an increased BSI risk index (-0.15, IQR = 0.48) as compared to patients that did not develop BSI (-0.33, IQR = 0.33). However, the difference was not statistically significant (Mann Whitney U test, p = 0.06) (Figure 10).

### METHODS

### Study patients and fecal sample collection.

Participants with non-Hodgkin's lymphoma were recruited in the hematology department of Nantes University Hospital, France. We excluded patients with a history of inflammatory bowel diseases, those exposed to probiotics, prebiotics or broad-spectrum antibiotics, and those administered nasal-tube feeding or parenteral nutrition in the month prior to initiation of the study. Participants received the same myeloablative conditioning regimen for 5 consecutive days, including high dose Carmustine (Bis-chloroethylnitrosourea), Etoposide, Aracytine and Melphalan, and HSCT occurred on the seventh day, as reported previously (73). Most of the participants received antibiotic prophylaxis before the conditioning therapy, including penicillin V and cotrimoxazole, which was stopped on the hospital inpatient admission (Table 1). Therefore, patients did not receive antibiotics during the chemotherapy procedure. BSI, the endpoint of the study, was assessed during inpatient HSCT hospitalization, following standard Centers for Disease Control and Prevention definitions of a laboratory-confirmed bloodstream infection (74). We collected a fecal sample from all participants. The fecal sample was collected on hospital inpatient admission (Day 0), prior to administration of the high-dose chemotherapy conditioning the transplant. After homogenization with a sterile spatula, 1 gram of stool was put into a sterile tube for subsequent molecular analysis. All samples were stored at -80°C until analysis.

### DNA Extraction and Purification.

The genomic DNA extraction procedure was based on the QIAamp® DNA Stool Minikit (Qiagen®). First, 200 mg of fecal sample were homogenized in 180 µL of lysozyme buffer and incubated for 30 min at 37°C. Mechanical lysis was obtained by adding 1.220 mL of ASL buffer and 300 mg of glass beads. The mixture was shaken using the Minibeadbeater-16 (Bead-beater Biospec Products®). The homogenized sample was heated at 95°C for 5 min and centrifuged for 1 min at 13,000 rpm. The supernatant was transferred into a 2 mL tube and an InhibitEX tablet was added. After dissolution, the sample was incubated for 1 min at room temperature and centrifuged for 6 min at 13,200 rpm. The supernatant was transferred into a 1.5 mL tube and centrifuged for 3 min at 13,200 rpm. Then, 200 µL of supernatant were mixed with 15 µL of proteinase K and 200 µL of AL buffer and incubated at 70°C for 10 min. 200 µL of ethanol were added and the solution was mixed. The complete lysate was applied to the column and centrifuged for 2 min at 13,200 rpm. After 2 washing processes with 500 µL of buffer AW1 and 500 µL of buffer AW2, DNA was eluted by adding 200 µL of buffer AE. DNA quality was assessed by gel electrophoresis and spectrophotometry measuring OD ratio 260/280 with the nanoDrop® ND-1000 (Thermo Fisher Scientific Inc®). The DNA aliquots were then stored at - 20°C.

### PCR Amplification of V5-V6 Region of Bacterial 16S rRNA Genes.

For each sample, we amplified 16S rRNA genes, using a primer set corresponding to primers 784F (AGGATTAGATACCCTGGTA) (SEQ ID NO:1) and 1061R (CRRCACGAGCTGACGAC) (SEQ ID NO:2), targeting the V5 and V6 hypervariable 16S rRNA gene region (∼280 nt region of the 16S rRNA gene) (75). The forward primer contained the sequence of the Titanium A adaptor (5'-CCATCTCATCCCTGCGTGTCTCCGACTCAG-3')(SEQ ID NO:3) and a barcode sequence. The reverse primer contained the sequence of Titanium B adaptor (5'-CCTATCCCCTGTGTGCCTTGGCAGTCTCAG-3') (SEQ ID NO:4). For each sample, a PCR mix of 100 µL was prepared containing PCR buffer, 2 u of KAPA HiFi Hotstart polymerase blend, and dNTPs (KAPA biosystems®), 300 nM primers (Eurogentec®), and 60 ng DNA. Thermal cycling consisted of an initial denaturing step at 95°C for 5 min, 25 cycles of denaturing at 98°C for 20 s, annealing at 56°C for 40 s, extension at 72°C for 20 s, and a final extension step at 72°C for 5 minutes. The amplicons were visualized using 1% agarose gels and GelGreen Nucleic Acid gel stain (Biotium®) in IX TEA buffer. The amplicons were purified using the Wizard® SV Gel and PCR Clean-up System (Promega®) according to the manufacturer's instructions.

### Amplicon Quantitation, Pooling, and Pyrosequencing.

Amplicon DNA concentrations were determined using the Quant-iT PicoGreen dsDNA reagent and kit (Invitrogen®) following the manufacturer's instructions. Assays were carried out using 10 µL of the cleaned PCR product in a total reaction volume of 200 µL in black, 96-well microtiter plates. Fluorescence was measured on a Perkin-Elmer Victor Plate reader using the 485/530 nm excitation/emission filter paired with a measurement time of 0.1 s. Following quantitation, the cleaned amplicons were combined in equimolar ratios in a single tube. The final pool of DNA was precipitated on ice for 45 min following the addition of 5 M NaCl (0.2 M final concentration) and two volumes of ice-cold 100% ethanol. The precipitated DNA was centrifuged at 7,800 × g for 40 min at 4°C, and the resulting pellet was washed with an equal volume of ice-cold 70% ethanol and centrifuged again at 7,800 × g for 20 min at 4 °C. The supernatant was removed, and the pellet was air dried for 10 min at room temperature and then resuspended in 100 µL nuclease-free water (Ambion®). The final concentration of the pooled DNA was determined using a NanoDrop spectrophotometer (Thermo Fisher Scientific Inc®). Pyrosequencing was carried out using primer A on a 454 Life Sciences Genome Sequencer FLX instrument (454 Life Sciences-Roche®) with titanium chemistry at at DNAVision (Charleroi, Belgium).

### Sequence Analysis.

The 16S rRNA raw sequences were analysed with the QIIME (Quantitative Insights Into Microbial Ecology) 1.8.0 software (76). Sequences were assigned to 97% ID OTUs by comparing them to the Greengenes reference database 13_8 (77).

We applied the following built-in functions of the QIIME pipeline. We used the beta_diversity_through_plots.py script to compute beta diversity metrics, which assess the differences in bacterial community composition between fecal samples of the patients who did or did not develop BSI. We represented beta diversity based on Unweighted UniFrac distances and visualized with Principal Coordinate Analysis (PCoA). We used the compare_categories.py script, which applied the Anosim method on the previously obtained dissimilarity matrices, and 999 random permutations of the residual under the reduced model, to determine whether communities differ significantly between fecal samples of patients who ultimately did or did not develop BSI. We used the alpha rarefaction.py script to compute alpha diversity metrics, which evaluated diversity within a sample and generated rarefaction curves. We used both non-phylogeny based metrics (Observed species, Chaol index, Shannon index) and phylogeny-based metrics (Phylogenetic Distance whole tree) to generate these metrics. We then tested differences in alpha diversity between fecal samples of patients who did or did not develop subsequent BSI with a Monte Carlo permuted t-test using the compare_alpha_diversity.py script. We used the supervised learning.py script and performed Random Forest classification with 500 trees and 10-fold cross-validation to obtain robust estimates of the generalization error and feature importances (14). We used the relative abundance in each sample of genera as features. The measure of the method's success is its ability to classify new samples as coming from one of the two groups (i.e., BSI or no BSI). Random Forests assign an importance score to each genus by estimating the increase in error caused by removing that genus from the set of predictors. Here, we considered a genus to be highly predictive if its average importance score was at least 0.001 as previously done (78, 79).

### Strain conventional identification.

Bacteria were stored at -70°C by using the cryovial bead preservation system (Microbank; Pro-Lab Diagnostics, Richmond Hill, Ontario, Canada). For identification, one bead was spread on a blood agar plate and incubated for 24h in aerobic conditions at 37°C. All isolates were identified at the species level by biochemical tests and confirmed by matrix-assisted laser desorption/ionization time-of-flight mass spectrometry with a VitekMS® mass spectrometer (bioMérieux SA, Marcy l'Etoile, France).

### Strain molecular identification.

DNA from clinical isolates was extracted using the InstaGene Matrix method (Bio-Rad Laboratories, Hercules, CA, USA). 16S rRNA gene amplification and sequencing were performed with different sets of primers: forward primer 27F, 5'-AGA GTT TGA TCM TGG CTC AG-3'(SEQ ID NO:5); reverse primer 1492R, 5'- TAC GGY TAC CTT GTT ACG ACT T-3'(SEQ ID NO:6) for PCR and forward primer 530F, 5'-GTG CCA GCM GCC GCG G-3'(SEQ ID NO:7); reverse primer 515R, 5'-ACC GCG GCK GCT GGC AC-3' (SEQ ID NO:8); forward primer 1114F, 5'-GCA ACG AGC GCA ACC C-3' (SEQ ID NO:9); reverse primer 1100R, 5'-GGG TTG CGC TCG TTG-3'(SEQ ID NO:10) for sequencing. PCR fragments were purified and sequenced using the ABI PRISM Big dye terminator v1.1 cycle sequencing ready reaction kit (Applied Biosystems, Courtaboeuf, France). The corresponding amplicons sequenced in both strands were compared with those in the Bioinformatics Bacteria Identification (BIBI) and BLAST databases. The rates of concordance between 16S rRNA gene sequences were based on results at the genus (≥97% similarity) and species (≥99% similarity) levels.

### Statistical analysis.

Quantitative data were reported as medians [25% Percentile-75% Percentile]. Qualitative data were reported as percentages [95% Confidence Interval]. We used PICRUSt, a computational approach to predict the functional composition of a metagenome using marker gene data (in this case the 16S rRNA gene) and a database of reference genomes. PICRUSt recaptures key findings from the Human Microbiome Project and predicts the abundance of gene families in host-associated and environmental communities with an accuracy of 90% on human intestinal communities (15). We used a linear model to find associations between clinical data (age, sex, type of antibiotic prophylaxis administered before admission, duration of pre-admission antibiotic prophylaxis, history of chemotherapy, and delay of previous chemotherapy) and taxa using code available at on the World Wide Web at github.com/danknights/mwas.

Moreover, we developed a BSI risk index corresponding to the difference between a patient's total relative abundance of taxa associated with protection from BSI and the patient's total relative abundance of taxa associated with development of a subsequent BSI. Relative abundances were arcsine square root transformed. We also evaluated biomarkers that should predict the risk of subsequent BSI based on relative abundance of differentially expressed taxa in pre-treatment fecal samples. We plotted receiving operating characteristic (ROC) curves and computed the area under the curve (AUC) values on a dataset containing 10 sets of predictions and corresponding labels obtained from 10-fold cross-validation using ROCR package in R (80).

### Citations for Example 1:

1. Tuncer HH, Rana N, Milani C, Darko A, Al-Homsi SA. Gastrointestinal and hepatic complications of hematopoietic stem cell transplantation. World J. Gastroenterol. WJG 2012;18(16):1851-1860.
2. Peterson DE, Bensadoun R-J, Roila F, ESMO Guidelines Working Group. Management of oral and gastrointestinal mucositis: ESMO Clinical Practice Guidelines. Ann. Oncol. 2011;22 Suppl 6:vi78-84.
3. Keefe DM et al. Updated clinical practice guidelines for the prevention and treatment of mucositis. Cancer 2007;109(5): 820-831.
4. Sonis ST. The pathobiology of mucositis. Nat. Rev. Cancer 2004;4(4):277-284.
5. Berg RD. Bacterial translocation from the gastrointestinal tract. Adv. Exp. Med. Biol. 1999;473:11-30.
6. Almyroudis NG et al. Pre- and post-engraftment bloodstream infection rates and associated mortality in allogeneic hematopoietic stem cell transplant recipients. Transpl. Infect. Dis. 2005;7(1):11-17.
7. Blennow O, Ljungman P, Sparrelid E, Mattsson J, Remberger M. Incidence, risk factors, and outcome of bloodstream infections during the pre-engraftment phase in 521 allogeneic hematopoietic stem cell transplantations. Transpl. Infect. Dis. 2014;16(1):106-114.
8. Mikulska M et al. Enterococcal bloodstream infection after hematopoietic stem cell transplant: experience of a center with a low prevalence of vancomycin-resistant enterococci. Clin. Infect. Dis. 2012;55(12):1744.
9. Elting LS, Rubenstein EB, Rolston KV, Bodey GP. Outcomes of bacteremia in patients with cancer and neutropenia: observations from two decades of epidemiological and clinical trials. Clin. Infect. Dis.. 1997;25(2):247-259.
10. Freifeld AG et al. Clinical practice guideline for the use of antimicrobial agents in neutropenic patients with cancer: 2010 update by the infectious diseases society of america. Clin. Infect. Dis. 2011;52(4):e56-93.
11. Klastersky J. Management of fever in neutropenic patients with different risks of complications. Clin. Infect. Dis. 2004;39 Suppl 1:S32-37.
12. Van Vliet MJ, Harmsen HJM, de Bont ESJM, Tissing WJE. The role of intestinal microbiota in the development and severity of chemotherapy-induced mucositis. PLoS Pathog. 2010;6(5):e1000879.
13. Taur Y et al. Intestinal domination and the risk of bacteremia in patients undergoing allogeneic hematopoietic stem cell transplantation. Clin. Infect. Dis. 2012;55(7):905-914.
14. Knights D, Costello EK, Knight R. Supervised classification of human microbiota. FEMS Microbiol. Rev. 2011;35(2):343-359.
15. Langille MGI et al. Predictive functional profiling of microbial communities using 16S rRNA marker gene sequences. Nat. Biotechnol. 2013;31(9):814-821.
16. Segata N et al. Metagenomic biomarker discovery and explanation. Genome Biol. 2011;12(6):R60.
17. Kashyap PC et al. Genetically dictated change in host mucus carbohydrate landscape exerts a diet-dependent effect on the gut microbiota. Proc. Natl. Acad. Sci. U. S. A. 2013; 110(42):17059-17064.
18. Kleiveland CR et al. The noncommensal bacterium Methylococcus capsulatus (Bath) ameliorates dextran sulfate (Sodium Salt)-Induced Ulcerative Colitis by influencing mechanisms essential for maintenance of the colonic barrier function. Appl. Environ. Microbiol. 2013;79(1):48-56.
19. Sperandio B et al. Virulent Shigella flexneri affects secretion, expression, and glycosylation of gel-forming mucins in mucus-producing cells. Infect. Immun. 2013;81(10):3632-3643.
20. Wrzosek L et al. Bacteroides thetaiotaomicron and Faecalibacterium prausnitzii influence the production of mucus glycans and the development of goblet cells in the colonic epithelium of a gnotobiotic model rodent. BMC Biol. 2013;11:61.
21. Sommer F et al. Altered mucus glycosylation in core 1 O-glycan-deficient mice affects microbiota composition and intestinal architecture. PloS One 2014;9(1):e85254.
22. An G et al. Increased susceptibility to colitis and colorectal tumors in mice lacking core 3-derived O-glycans. J. Exp. Med. 2007;204(6):1417-1429.
23. Koropatkin NM, Cameron EA, Martens EC. How glycan metabolism shapes the human gut microbiota. Nat. Rev. Microbiol. 2012;10(5):323-335.
24. McNeil NI. The contribution of the large intestine to energy supplies in man. Am. J. Clin. Nutr. 1984;39(2):338-342.
25. Eloe-Fadrosh EA, Rasko DA. The human microbiome: from symbiosis to pathogenesis. Annu. Rev. Med. 2013;64:145-163.
26. Gevers D et al. The treatment-naive microbiome in new-onset Crohn's disease. Cell Host Microbe 2014;15(3):382-392.
27. Lyte M, Vulchanova L, Brown DR. Stress at the intestinal surface: catecholamines and mucosa-bacteria interactions. Cell Tissue Res. 2011;343(1):23-32.
28. Faure M et al. Specific amino acids increase mucin synthesis and microbiota in dextran sulfate sodium-treated rats. J. Nutr. 2006;136(6):1558-1564.
29. Vázquez-Castellanos JF et al. Altered metabolism of gut microbiota contributes to chronic immune activation in HIV-infected individuals. Mucosal Immunol. [published online ahead of print: November 19, 2014]; doi:10.1038/mi.2014.107
30. Peterson JW, Boldogh I, Popov VL, Saini SS, Chopra AK. Anti-inflammatory and antisecretory potential of histidine in Salmonella-challenged mouse small intestine. Lab. Investig. J. Tech. Methods Pathol. 1998;78(5):523-534.
31. Son DO, Satsu H, Shimizu M. Histidine inhibits oxidative stress- and TNF-alpha-induced interleukin-8 secretion in intestinal epithelial cells. FEBS Lett. 2005;579(21):4671-4677.
32. Quirino IEP, Correia MITD, Cardoso VN. The impact of arginine on bacterial translocation in an intestinal obstruction model in rats. Clin. Nutr. Edinb. Scotl. 2007;26(3):335-340.
33. Tan B et al. L-Arginine stimulates proliferation and prevents endotoxin-induced death of intestinal cells. Amino Acids 2010;38(4):1227-1235.
34. Viana ML et al. Pretreatment with arginine preserves intestinal barrier integrity and reduces bacterial translocation in mice. Nutr. 2010;26(2):218-223.
35. Yeh C-L, Yeh S-L, Lin M-T, Chen W-J. Effects of arginine-enriched total parenteral nutrition on inflammatory-related mediator and T-cell population in septic rats. Nutr. 2002;18(7-8):631-635.
36. Ding L-A, Li J-S. Effects of glutamine on intestinal permeability and bacterial translocation in TPN-rats with endotoxemia. World J. Gastroenterol. 2003;9(6):1327-1332.
37. Van Zwol A, Neu J, van Elburg RM. Long-term effects of neonatal glutamine-enriched nutrition in very-low-birth-weight infants. Nutr. Rev. 2011;69(1):2-8.
38. Clark EC et al. Glutamine deprivation facilitates tumour necrosis factor induced bacterial translocation in Caco-2 cells by depletion of enterocyte fuel substrate. Gut 2003;52(2):224-230.
39. Edgar RC. Search and clustering orders of magnitude faster than BLAST. Bioinforma. 2010;26(19):2460-2461.
40. Taur Y et al. The effects of intestinal tract bacterial diversity on mortality following allogeneic hematopoietic stem cell transplantation. Blood 2014;124(7):1174-1182.
41. Human Microbiome Project Consortium. Structure, function and diversity of the healthy human microbiome. Nature 2012;486(7402):207-214.
42. Hansen R et al. Microbiota of de-novo pediatric IBD: increased Faecalibacterium prausnitzii and reduced bacterial diversity in Crohn's but not in ulcerative colitis. Am. J. Gastroenterol. 2012;107(12):1913-1922.
43. Manichanh C et al. Reduced diversity of faecal microbiota in Crohn's disease revealed by a metagenomic approach. Gut 2006;55(2):205-211.
44. Craven M et al. Inflammation drives dysbiosis and bacterial invasion in murine models of ileal Crohn's disease. PloS One 2012;7(7):e41594.
45. Ubeda C et al. Intestinal microbiota containing Barnesiella species cures vancomycin-resistant Enterococcus faecium colonization. Infect. Immun. 2013;81(3):965-973.
46. Marin M et al. Bloodstream infections in neutropenic patients with cancer: differences between patients with haematological malignancies and solid tumours. J. Infect. 2014;69(5):417-423.
47. Montassier E, Batard E, Gastinne T, Potel G, de La Cochetière MF. Recent changes in bacteremia in patients with cancer: a systematic review of epidemiology and antibiotic resistance. Eur. J. Clin. Microbiol. Infect. Dis. 2013;32(7):841-850.
48. Mutlu EA et al. A compositional look at the human gastrointestinal microbiome and immune activation parameters in HIV infected subjects. PLoS Pathog. 2014;10(2):e1003829.
49. Dinh DM et al. Intestinal Microbiota, Microbial Translocation, and Systemic Inflammation in Chronic HIV Infection. J. Infect. Dis. 2015;211(1):19-27.
50. Zenewicz LA et al. IL-22 deficiency alters colonic microbiota to be transmissible and colitogenic. J. Immunol. 1950 2013;190(10):5306-5312.
51. Morgan XC et al. Dysfunction of the intestinal microbiome in inflammatory bowel disease and treatment. Genome Biol. 2012;13(9):R79.
52. Sokol H et al. Faecalibacterium prausnitzii is an anti-inflammatory commensal bacterium identified by gut microbiota analysis of Crohn disease patients. Proc. Natl. Acad. Sci. U. S. A. 2008;105(43):16731-16736.
53. Giannelli V et al. Microbiota and the gut-liver axis: bacterial translocation, inflammation and infection in cirrhosis. World J. Gastroenterol. 2014;20(45):16795-16810.
54. Fuentes S et al. Reset of a critically disturbed microbial ecosystem: faecal transplant in recurrent Clostridium difficile infection. ISME J. 2014;8(8):1621-1633.
55. Goodrich JK et al. Human genetics shape the gut microbiome. Cell 2014;159(4):789-799.
56. Rajilić-Stojanović M, Shanahan F, Guarner F, de Vos WM. Phylogenetic analysis of dysbiosis in ulcerative colitis during remission. Inflamm. Bowel Dis. 2013;19(3):481-488.
57. Lam YY et al. Increased gut permeability and microbiota change associate with mesenteric fat inflammation and metabolic dysfunction in diet-induced obese mice. PloS One 2012;7(3):e34233.
58. Wong JMW, de Souza R, Kendall CWC, Emam A, Jenkins DJA. Colonic health: fermentation and short chain fatty acids. J. Clin. Gastroenterol. 2006;40(3):235-243.
59. Hamer HM et al. Review article: the role of butyrate on colonic function. Aliment. Pharmacol. Ther. 2008;27(2):104-119.
60. Hamer HM et al. Butyrate modulates oxidative stress in the colonic mucosa of healthy humans. Clin. Nutr. 2009;28(1):88-93.
61. Papa E et al. Non-invasive mapping of the gastrointestinal microbiota identifies children with inflammatory bowel disease. PloS One 2012;7(6):e39242.
62. Jalanka-Tuovinen J et al. Faecal microbiota composition and host-microbe cross-talk following gastroenteritis and in postinfectious irritable bowel syndrome. Gut 2014;63(11):1737-1745.
63. Rey FE et al. Metabolic niche of a prominent sulfate-reducing human gut bacterium. Proc. Natl. Acad. Sci. U. S. A. 2013;110(33):13582-13587.
64. Bhargava P, Mowry EM. Gut microbiome and multiple sclerosis. Curr. Neurol. Neurosci. Rep. 2014;14(10):492.
65. Satokari R et al. Fecal transplantation treatment of antibiotic-induced, noninfectious colitis and long-term microbiota follow-up. Case Rep. Med. 2014;2014:913867.
66. Rigsbee L et al. Quantitative profiling of gut microbiota of children with diarrhea-predominant irritable bowel syndrome. Am. J. Gastroenterol. 2012;107(11):1740-1751.
67. Bibbò S et al. Role of microbiota and innate immunity in recurrent Clostridium difficile infection. J. Immunol. Res. 2014;2014:462740.
68. Wang W et al. Increased proportions of Bifidobacterium and the Lactobacillus group and loss of butyrate-producing bacteria in inflammatory bowel disease. J. Clin. Microbiol. 2014;52(2):398-406.
69. Palm NW et al. Immunoglobulin A coating identifies colitogenic bacteria in inflammatory bowel disease. Cell 2014;158(5):1000-1010.
70. Marchesi JR et al. Towards the human colorectal cancer microbiome. PloS One 2011;6(5):e20447.
71. Zackular JP et al. The gut microbiome modulates colon tumorigenesis. mBio 2013;4(6):e00692-00613.
72. Pamer EG. Fecal microbiota transplantation: effectiveness, complexities, and lingering concerns. Mucosal Immunol. 2014;7(2):210-214.
73. Montassier E et al. 16S rRNA gene pyrosequencing reveals shift in patient faecal microbiota during high-dose chemotherapy as conditioning regimen for bone marrow transplantation. Microb. Ecol. 2014;67(3):690-699.
74. Centers for Disease Control and Prevention. Central Line-Associated Bloodstream Infection (CLABSI) Event. Available at: http://www.cdc.gov/nhsn/pdfs/pscmanual/4psc_clabscurrent.pdf. Accessed 23 May 2014
75. Andersson AF et al. Comparative analysis of human gut microbiota by barcoded pyrosequencing. PloS One 2008;3(7):e2836.
76. Caporaso JG et al. QIIME allows analysis of high-throughput community sequencing data. Nat. Methods 2010;7(5):335-336.
77. DeSantis TZ et al. Greengenes, a chimera-checked 16S rRNA gene database and workbench compatible with ARB. Appl. Environ. Microbiol. 2006;72(7):5069-5072.
78. Lozupone CA et al. Alterations in the gut microbiota associated with HIV-1 infection. Cell Host Microbe 2013;14(3):329-339.
79. Yatsunenko T et al. Human gut microbiome viewed across age and geography. Nature 2012;486(7402):222-227.
80. Sing T, Sander O, Beerenwinkel N, Lengauer T. ROCR: visualizing classifier performance in R. Bioinforma. 2005;21(20):3940-3941.

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

Unless otherwise indicated, all numbers expressing quantities of components, molecular weights, and so forth used in the specification and claims are to be understood as being modified in all instances by the term "about." Such term "about" shall include +/- 10, 9, 8, 7, 6, 5, 4, 3, 2, or 1% of the stated value.

Notwithstanding that the numerical ranges and parameters setting forth the broad scope of the invention are approximations, the numerical values set forth in the specific examples are reported as precisely as possible. All numerical values, however, inherently contain a range necessarily resulting from the standard deviation found in their respective testing measurements.

All headings are for the convenience of the reader and should not be used to limit the meaning of the text that follows the heading, unless so specified.

## Claims

1. A method for determining the relative abundance of total bacteria in a sample, the method comprising:
(A) providing a fecal sample from a subject before the subject receives chemotherapy;
(B) determining the relative abundance of total bacteria in the fecal sample;
(C) detecting in the fecal sample bacteria that correlate with developing a bloodstream infection after chemotherapy and calculating the relative abundances of bacteria that correlate with developing a bloodstream infection after chemotherapy; and
(D) detecting in the fecal sample bacteria that correlate with not developing a bloodstream infection after chemotherapy and calculating the relative abundances of bacteria that correlate with not developing a bloodstream infection after chemotherapy;
wherein:
(i) the detecting of (C) comprises an assay that detects a member of family Erysipelotrichaceae, a member of genus *Lactobacillus,* a member of genus *Eggerthella*, a member of genus *Veillonella*, or a combination thereof; and/or
(ii) the detecting of (D) comprises an assay that detects a member of the order RF39 in the class Mollicutes, a member of the order Clostridiales, a member of family Barnesiellaceae, a member of family Coriobacteriaceae, a member of family Rikenellaceae, a member of genus *Butyricimonas,* a member of genus *Faecalibacterium,* such as *Faecalibacterium prausnitzii,* a member of genus *Oscillospira,* a member of family Christensenellaceae such as genus *Christensenella,* a member of genus *Dehalobacterium,* a member of genus *Desulfovibrio,* a member of genus *Sutterella,* a member of genus *Methanobrevibacter*, a member of genus *Oxalobacter*, or a combination thereof,
wherein reference to a taxonomic group above species level excludes all lower taxonomic members of that group that are otherwise specifically identified.

2. The method of claim 1 wherein:
(i) the detecting of (C) comprises an assay that detects a member of family Erysipelotrichaceae, a member of genus *Lactobacillus,* a member of genus *Eggerthella*, a member of genus *Veillonella*, or a combination thereof; and
(ii) the detecting of (D) comprises an assay that detects a member of the order RF39 in the class Mollicutes, a member of the order Clostridiales, a member of family Barnesiellaceae, a member of family Coriobacteriaceae other than the genus *Eggerthella,* a member of family Rikenellaceae, a member of genus *Butyricimonas,* a member of genus *Faecalibacterium,* such as *Faecalibacterium prausnitzii,* a member of genus *Oscillospira,* a member of family Christensenellaceae such as genus *Christensenella,* a member of genus *Dehalobacterium,* a member of genus *Desulfovibrio,* a member of genus *Sutterella,* a member of genus *Methanobrevibacter*, a member of genus *Oxalobacter*, or a combination thereof.

3. The method of any of claims 1-2 further comprising:
(E) generating a bloodstream infection risk index score; and
(F) determining whether the subject has a risk of bloodstream infection in accordance with the result of (E), optionally wherein a subject with a bloodstream risk index score that is greater than a threshold is at higher risk of a blood stream infection after undergoing chemotherapy, further optionally wherein the threshold is between -1 and 1 is at higher risk of a blood stream infection after undergoing chemotherapy.

4. The method of any of claims 1-2 wherein determining the relative abundances in the fecal sample comprises performing a quantitative polymerase chain reaction, or wherein determining the relative abundances in the fecal sample comprises performing a high-throughput DNA sequencing.

5. The method of any of claims 1-2 wherein
(i) the determining of (B) comprises analysis of a 16S rRNA variable region of the bacteria in the fecal sample, wherein the 16S rRNA variable region comprises V1, V2, V3, V4, V5, V6, V7, V8, or V9, or a combination thereof, or
(ii) the detecting of (C), (D), or both (C) and (D) comprises analysis of the 16S rRNA region of the bacteria in the fecal sample.

6. The method of any of claims 1-2 wherein the chemotherapy is in preparing the subject for a hematopoietic stem cell transplantation, or wherein the subject undergoes a hematopoietic stem cell transplantation after the chemotherapy, or wherein the chemotherapy of (A) comprises a non-myeloablative regimen or a myeloablative regimen.

## Patentansprüche

1. Verfahren zum Bestimmen der relativen Häufigkeit von Gesamtbakterien in einer Probe, wobei das Verfahren Folgendes umfasst:
(A) Bereitstellen einer Stuhlprobe von einem Subjekt, bevor das Subjekt eine Chemotherapie erhält;
(B) Bestimmen der relativen Häufigkeit von Gesamtbakterien in der Stuhlprobe;
(C) Nachweisen von Bakterien in der Stuhlprobe, die mit einem Entwickeln einer Blutkreislaufinfektion nach der Chemotherapie korrelieren, und Berechnen der relativen Häufigkeit von Bakterien, die mit dem Entwickeln einer Blutkreislaufinfektion nach der Chemotherapie korrelieren; und
(D) Nachweisen von Bakterien in der Stuhlprobe, die mit einem Nichtentwickeln einer Blutkreislaufinfektion nach der Chemotherapie korrelieren, und Berechnen der relativen Häufigkeit von Bakterien, die mit dem Nichtentwickeln einer Blutkreislaufinfektion nach der Chemotherapie korrelieren;
wobei:
(i) das Nachweisen von (C) einen Assay umfasst, der ein Mitglied der Familie Erysipelotrichaceae, ein Mitglied der Gattung *Lactobacillus,* ein Mitglied der Gattung *Eggerthella,* ein Mitglied der Gattung *Veillonella* oder eine Kombination davon nachweist; und/oder
(ii) das Nachweisen von (D) einen Assay umfasst, der ein Mitglied der Ordnung RF39 in der Klasse Mollicutes, ein Mitglied der Ordnung Clostridiales, ein Mitglied der Familie Barnesiellaceae, ein Mitglied der Familie Coriobacteriaceae, ein Mitglied der Familie Rikenellaceae, ein Mitglied der Gattung *Butyricimonas,* ein Mitglied der Gattung *Faecalibacterium,* wie etwa *Faecalibacterium prausnitzii,* ein Mitglied der Gattung *Oscillospira,* ein Mitglied der Familie Christensenellaceae, wie etwa die Gattung *Christensenella,* ein Mitglied der Gattung *Dehalobacterium,* ein Mitglied der Gattung *Desulfovibrio,* ein Mitglied der Gattung *Sutterella,* ein Mitglied der Gattung *Methanobrevibacter,* ein Mitglied der Gattung *Oxalobacter* oder eine Kombination davon nachweist,
wobei eine Bezugnahme auf eine taxonomische Gruppe über dem Artenniveau alle niedrigeren taxonomischen Mitglieder dieser Gruppe ausschließt, die ansonsten speziell identifiziert werden.

2. Verfahren nach Anspruch 1, wobei:
(i) das Nachweisen von (C) einen Assay umfasst, der ein Mitglied der Familie Erysipelotrichaceae, ein Mitglied der Gattung *Lactobacillus,* ein Mitglied der Gattung *Eggerthella,* ein Mitglied der Gattung *Veillonella* oder eine Kombination davon nachweist; und
(ii) das Nachweisen von (D) einen Assay umfasst, der ein Mitglied der Ordnung RF39 in der Klasse Mollicutes, ein Mitglied der Ordnung Clostridiales, ein Mitglied der Familie Bamesiellaceae, ein Mitglied der Familie Coriobacteriaceae außer der Gattung *Eggerthella,* ein Mitglied der Familie Rikenellaceae, ein Mitglied der Gattung *Butyricimonas,* ein Mitglied der Gattung *Faecalibacterium,* wie etwa *Faecalibacterium prausnilzii,* ein Mitglied der Gattung *Oscillospira,* ein Mitglied der Familie Christensenellaceae, wie etwa die Gattung *Christensenella,* ein Mitglied der Gattung *Dehalobacterium,* ein Mitglied der Gattung *Desulfovibrio,* ein Mitglied der Gattung *Sutterella,* ein Mitglied der Gattung *Methanobrevibacter,* ein Mitglied der Gattung *Oxalobacter* oder eine Kombination davon nachweist.

3. Verfahren nach einem der Ansprüche 1-2, das ferner Folgendes umfasst:
(E) Generieren eines Blutkreislaufinfektionsrisikoindexwertes; und
(F) Bestimmen, ob das Subjekt ein Risiko einer Blutkreislaufinfektion gemäß dem Ergebnis von (E) aufweist, optional wobei ein Subjekt mit einem Blutkreislaufinfektionsrisikoindexwert, der über einer Schwelle liegt, ein höheres Risiko einer Blutkreislaufinfektion hat, nachdem es sich einer Chemotherapie unterzogen hat, ferner optional wobei die Schwelle zwischen -1 und 1 ein höheres Risiko für eine Blutkreislaufinfektion nach der Chemotherapie bedeutet.

4. Verfahren nach einem der Ansprüche 1-2, wobei das Bestimmen der relativen Häufigkeit in der Stuhlprobe ein Durchführen einer quantitativen Polymerasekettenreaktion umfasst oder wobei das Bestimmen der relativen Häufigkeit in der Stuhlprobe das Durchführen einer DNA-Sequenzierung mit hohem Durchsatz umfasst.

5. Verfahren nach einem der Ansprüche 1-2, wobei
(i) das Bestimmen von (B) eine Analyse einer variablen 16S-rRNA-Region der Bakterien in der Stuhlprobe umfasst, wobei die variable 16S-rRNA-Region V1, V2, V3, V4, V5, V6, V7, V8 oder V9 oder eine Kombination davon umfasst, oder
(ii) das Nachweisen von (C), (D) oder sowohl (C) als auch (D) die Analyse der 16S-rRNA-Region der Bakterien in der Stuhlprobe umfasst.

6. Verfahren nach einem der Ansprüche 1-2, wobei die Chemotherapie darin besteht, das Subjekt auf eine blutbildende Stammzelltransplantation vorzubereiten, oder wobei das Subjekt nach der Chemotherapie einer blutbildenden Stammzelltransplantation unterzogen wird oder wobei die Chemotherapie von (A) einen nichtmyeloablativen Therapieplan oder einen myeloablativen Therapieplan umfasst.

## Revendications

1. Procédé de détermination de l'abondance relative de bactéries totales dans un échantillon, le procédé comprenant :
(A) la fourniture d'un échantillon fécal à partir d'un sujet avant que le sujet reçoive une chimiothérapie ;
(B) la détermination de l'abondance relative de bactéries totales dans l'échantillon fécal ;
(C) la détection dans l'échantillon fécal de bactéries qui sont en corrélation avec le fait de développer une bactériémie après une chimiothérapie et le calcul des abondances relatives de bactéries qui sont en corrélation avec le fait de développer une bactériémie après une chimiothérapie ; et
(D) la détection dans l'échantillon fécal de bactéries qui sont en corrélation avec le fait de ne pas développer une bactériémie après une chimiothérapie et le calcul des abondances relatives de bactéries en corrélation avec le fait de ne pas développer une bactériémie après une chimiothérapie ;
dans lequel :
(i) la détection de (C) comprend un dosage qui détecte un membre de la famille des Erysipelotrichaceae, un membre du genre *Lactobacillus*, un membre du genre *Eggerthella*, un membre du genre *Veillonella*, ou leur combinaison ; et/ou
(ii) la détection de (D) comprend un dosage qui détecte un membre de l'ordre RF39 dans la classe des Mollicutes, un membre de l'ordre des Clostridiales, un membre de la famille des Barnesiellaceae, un membre de la famille des Coriobacteriaceae, un membre de la famille des Rikenellaceae, un membre du genre *Butyricimonas*, un membre du genre *Faecalibacterium*, tel que *Faecalibacterium prausnitzii,* un membre du genre *Oscillospira*, un membre de la famille des Christensenellaceae tel que le genre *Christensenella*, un membre du genre *Dehalobacterium*, un membre du genre *Desulfovibrio*, un membre du genre *Sutterella,* un membre du genre *Methanobrevibacter,* un élément du genre *Oxalobacter*, ou leur combinaison,
dans lequel la référence à un groupe taxonomique au-dessus du niveau de l'espèce exclut tous les membres taxonomiques inférieurs de ce groupe qui sont par ailleurs spécifiquement identifiés.

2. Procédé selon la revendication 1, dans lequel :
(i) la détection de (C) comprend un dosage qui détecte un membre de la famille des Erysipelotrichaceae, un membre du genre *Lactobacillus*, un membre du genre *Eggerthella*, un membre du genre *Veillonella*, ou leur combinaison ; et
(ii) la détection de (D) comprenant un dosage qui détecte un membre de l'ordre RF39 dans la classe des Mollicutes, un membre de l'ordre des Clostridiales, un membre de la famille des Bamesiellaceae, un membre de la famille des Coriobacteriaceae autre que le genre *Eggerthella,* un membre de la famille des Rikenellaceae, un membre du genre *Butyricimonas,* un membre du genre *Faecalibacterium*, tel que *Faecalibacterium prausnilzii,* un membre du genre *Oscillospira*, un membre de la famille des Christensenellaceae tels que le genre *Christensenella*, un membre du genre *Dehalobacterium*, un membre du genre *Desulfovibrio*, un membre du genre *Sutterella,* un membre du genre *Methanobrevibacter,* un membre du genre *Oxalobacter*, ou leur combinaison.

3. Procédé selon l'une quelconque des revendications 1 à 2, comprenant en outre :
(E) la génération d'un score d'indice de risque de bactériémie ; et
(F) la détermination du fait de savoir si le sujet présente un risque de bactériémie conformément au résultat de (E), éventuellement dans laquelle le sujet dont le score d'indice de risque de bactériémie qui est supérieur à un seuil est davantage susceptible de développer une bactériémie après avoir subi une chimiothérapie, en outre éventuellement dans laquelle un sujet dont le seuil est compris entre -1 et 1 est davantage susceptible de développer une bactériémie après avoir subi une chimiothérapie.

4. Procédé selon l'une quelconque des revendications 1 à 2, la détermination des abondances relatives dans l'échantillon fécal comprenant la réalisation d'une réaction en chaîne par polymérase quantitative, ou la détermination des abondances relatives dans l'échantillon fécal comprenant la réalisation d'un séquençage de l'ADN à haut débit.

5. Procédé selon l'une quelconque des revendications 1 à 2, dans lequel
(i) la détermination de (B) comprend l'analyse d'une région variable d'ARNr 16S des bactéries dans l'échantillon fécal, la région variable d'ARNr 16S comprenant V1, V2, V3, V4, V5, V6, V7, V8 ou V9, ou leur combinaison, ou
(ii) la détection de (C), de (D) ou de (C) et (D) comprend l'analyse de la région d'ARNr 16S des bactéries dans l'échantillon fécal.

6. Procédé selon l'une quelconque des revendications 1 à 2, la chimiothérapie servant à préparer le sujet à une transplantation de cellules souches hématopoïétiques, ou le sujet subissant une transplantation de cellules souches hématopoïétiques après la chimiothérapie, ou la chimiothérapie de (A) comprenant un conditionnement non myéloablatif ou un conditionnement myéloablatif.
